# EUROPEAN PATENT SPECIFICATION

(11) **EP 2 978 455 B1**
(45) Date of publication and mention of the grant of the patent: **04.01.2017**
(21) Application number: 14710891.4
(22) Date of filing: 18.03.2014
(51) Int. Cl.: A61K 49/10, A61K 9/107, A61K 49/18, A61B 5/055, G01R 33/56

(54) **SEMIFLUOROCARBON COMPOUND CONTAINING CONTRAST AGENT**
SEMI-FLUORKOHLENSTOFF-VERBINDUNG MIT KONTRASTMITTEL
COMPOSÉ SEMI-FLUOCARBURE CONTENANT UN AGENT DE CONTRASTE

(30) Priority: 25.03.2013 EP 13160868
(43) Date of publication of application: 03.02.2016
(73) Proprietor: B. Braun Melsungen AG, 34212 Melsungen (DE)
(72) Inventor: KELLER, Thorsten, 76777 Neupotz (DE); RÖTHLEIN, Doris, 34121 Kassel (DE); SCHMITT, Jürgen, 35274 Kirchhain (DE); DIETRICH, Thore, 13469 Berlin (DE); STAWOWY, Philipp, 10623 Berlin (DE); FLECK, Eckart, 14193 Berlin (DE)
(74) Representative: dompatent von Kreisler Selting Werner - Partnerschaft von Patent- und Rechtsanwälten mbB
(86) International application number: PCT/EP2014/055398
(87) International publication number: WO 2014/154531

(56) References cited:
- AHRENS E. T. ET AL.: "In vivo imaging platform for tracking immunotherapeutic cells", NATURE BIOTECHNOLOGY, vol. 23, no. 8, 24 July 2005 (2005-07-24), pages 983-987, XP002700840, cited in the application
- Mayenfels F. G.: "Fluorcarbonhaltige Nanoemulsionen zur Anwendung in der 1H/19F-Magnetresonanztomographie", 5 July 2012 (2012-07-05), Albert-Ludwigs Universität,, Inauguraldissertation, Freiburg i. Br., XP002700841, Page 10; Page 50, line 2; page 63, 3rd paragraph; paragraph bridging pages 196 and 197.

## Description

The present invention relates to an aqueous emulsion comprising a specific semifluorocarbon compound, a mid-chain triglyceride (MCT) and an emulsifier as well as the use of the emulsion as a contrast agent or contrast enhancing agent. Further, the invention relates to the use of said emulsion for the diagnostic detection of inflammatory pathological conditions using MR imaging.

Inflammatory diseases are by far the most important causes of morbidity and mortality worldwide. While there are effective diagnostic and therapeutic methods for acute inflammatory diseases (predominantly caused by pathogens) in many cases, the diagnosis of chronic inflammatory diseases is mostly difficult, and the therapy thereof is limited to symptomatic measures. Non-invasive imaging methods, such as echocardiography, computer tomography and nuclear magnetic resonance spectroscopy, provide detailed anatomic information and are thus valuable tools for evaluating the function of organs. However, with none of the methods mentioned has it been possible to date to detect inflammatory processes unambiguously with high spatial resolution.

In Nature Biotechnology Vol. 23, No. 8, August 2005, p. 983-987, Arens et al. describe methods for tracking immunotherapeutic cells. By means of magnetic resonance imaging (MRI) of perfluoro-15-crown-5 ether, it is tried to detect cells in vivo that possess immunotherapeutic relevance. They used dendritic cells charged ex vivo with perfluoro-15-crown-5 ether. Subsequently, their fate after injection into mouse tissue or intravenous administration to mice was examined in vivo. WO 2005/072780 A2 and WO 03/075747 A2 relate to similar examinations. In Medica Mundi 47/1, April 2003, p. 34-39, G. M. Lanza et al. describe the use of paramagnetic nanoparticles for the targeting of cells. In this method, the paramagnetic nanoparticles are employed to produce signal extinction in the ¹H magnetic resonance image. Vehicles charged with ¹⁹F were employed as probes.

WO-A-2005/072780 relates to the ex vivo labeling of isolated cells with perfluoro-carbons. It is disclosed that isolated cells labeled with perfluorocarbons ex vivo are suitable for studying cell migrations in the organism after injection.

Oku, Naoto et al. (Biochimica et Biophysica Acta 1280 (1996), 149-154) relates to the use of liposomes charged with ¹⁸F-labeled glucose for studying "liposomal trafficking". The studies were performed by means of PET diagnostics.

US 2009/0280055 A1 discloses the use of perfluoroctylbromide for diagnostic purposes using imaging methods.

It is the object of the present invention to provide a method which enables the visualization of specifically pathological conditions, such as inflammatory processes, by means of imaging methods, and thus the recognition of the affected regions.

A further object of the present invention is the provision of fluorocarbon based diagnostic agents for the specific diagnosis of cardiovascular diseases, in particular diagnosis of inflammatory processes of the cardiovascular system, and more specifically for the diagnosis of Myocarditis or inflammatory cardiomyopathy.

A further object of the invention is the provision of a biocompatible oil in water emulsion which can be used for the magnetic resonance spectroscopy and which is storage stable even under stress conditions such as the sterilization.

A first embodiment of the present invention is an aqueous emulsion comprising
a) a semifluorinated compound of formula I:

   CF₃-(CF₂)ₓ-(CH₂)_{y}-CH₃ (I)

   wherein x is an integer ranging from 1 to 8 and y is an integer ranging from 2 to 10,
b) a triglyceride which is miscible with the semifluorinated compound at 20 °C; and
c) an emulsifier.

A further specific embodiment of the invention is an aqueous emulsion comprising
a) a semifluorinated compound of formula I:

   CF₃-(CF₂)ₓ-(CH₂)_{y}-CH₃ (I)

   wherein x is an integer ranging from 1 to 8 and y is an integer ranging from 2 to 10,
b) a mid-chain triglyceride (MCT) and
c) an emulsifier.

The emulsion of the invention is preferably an oil in water emulsion which mandatorily comprises a semifluorinated compound.

Semifluorinated compounds within the meaning of the present invention are semifluorinated alkanes. The semifluorinated alkanes comprise a fluorocarbon and a hydrocarbon moiety. Commonly, semifluorinated alkanes follow the following nomenclatures: FXHY wherein X represents the number of carbon atoms which are substituted by fluoro atoms and Y represents the number of carbon atoms which are substituted with hydrogen atoms. For example perfluorohexyloctane is represented by the formula: F6H8.

F6H8 is shown in the following formula:

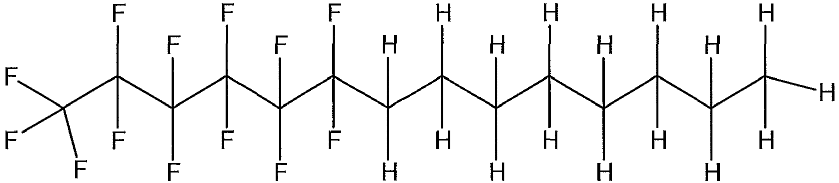

Semifluorinated compounds which are suitable for the emulsions of the present invention are reflected in formula (I):

CF₃-(CF₂)ₓ-(CH₂)_{y}-CH₃ (I)

wherein x is an integer ranging from 1 to 8 and y is an integer ranging from 1 to 10.

In one embodiment x in formula (I) ranges from 2 to 6, preferably 3 to 6, more preferably 4 to 6 and especially x is 5.

In a further preferred embodiment y in formula (I) is ranging from 2 to 9, preferably 3 to 8, more preferably 5 to 8, further preferably 6 to 8 and especially y is 7.

Good results in terms of imaging and stability of the emulsion can be achieved with semifluorinated compounds of formula (I) wherein y is greater than x.

Especially preferred semifluorinated compounds are perfluorohexyloctane (F6H8) and/or perfluorbutylpentane (F4H5).

A further component of the invention is a triglyceride which is miscible with the semifluorinated compound at 20 °C (hereinafter also compound b)).

Within the meaning of the present invention the triglyceride (component b)) is miscible in the semifluorinated compound (component a)) if at a temperature of 20 °C and after mixing and subsequent storing at 20 °C for 24 hour the component a) and component b) do not form separate continuous phases.

In a preferred embodiment the component b) is miscible in component a) in any ratio. In particular, component a) and component b) are miscible in a weight ratio of component a) to component b) from 1:20 to 1:0.7, preferably 1:15 to 1:0.8, more preferably 1:10 to 1:0.9, further preferably 1:4 to 1:1, especially 1:2 to 1:1.

In an alternative embodiment the weight ratio of component a) to component b) is from 1:20 to 1:0.20, preferably 1:15 to 1:0.25, more preferably 1:10 to 1:0.30, further preferably 1:4 to 1:0.35, especially 1:2 to 1:0.4.

In a preferred embodiment the triglyceride which is miscible with the semifluorinated compound is a mid-chain triglyceride (MCT).

In a specific embodiment the weight ratio of component a), preferably F6H8, to MCT ranges from 4:1 to 1:4, especially 3:2 to 2:3.

The oil of the aqueous emulsion described herein preferably comprises medium chain triglycerides. "Medium chain triglycerides" (MCTs) are a class of triglyceride oil that can be either naturally derived or synthetic. MCTs are formed from fatty acids of 6 to 14 carbons, preferably 6 to 12 carbons, especially 8 to 10 carbons, in length. The MCT can contain caprylic acid (for example, in an amount of about 50% to about 80% by weight of the MCT), an 8-carbon saturated FA (8:0). The MCT can contain capric acid (for example, in an amount of about 20% to about 50% by weight of the MCT), a 10-carbon saturated FA (10:0). For example, the medium-chain triglycerides can contain triglycerides of caprylic acid and capric acid, in an amount of at least 90% by weight of the medium-chain triglycerides. The description of the MCT for use in this disclosure can, for example, meet the requirements of EP monograph 0868, entitled "Triglycerides, Medium Chain" (Triglycerida saturate media) (EP 0868, 2008).

According to a further aspect of the invention the mid chain triglyceride (MCT) is glycerol which is esterified with carboxylic acids as specified in the following: caproic acid in an amount of 2 wt.-% or less;
caprylic acid in an amount ranging from about 50 to about 80 wt.-%;
capric acid in an amount ranging from about 20 to about 50 wt.-%;
lauric acid in an amount of 3 wt.-% or less;
myristic acid in an amount of 1 wt.-% or less, wherein the weight-% (wt.-%) is based on the total weight of the fatty acids.

The medium-chain triglycerides (MCT) are excellent solvents for the semifluorinated compounds. According to the invention one or more MCT's can be used. MCT is commercially available as for example Miglyol 812 (SASOL GmbH Germany), or CRODAMOL GTCC-PN (Croda Inc, New Jersey).

According to another preferred embodiment of the present invention the emulsion comprises an MCT which is consisting of glycerol which is esterified with fatty acids comprising at least 50 wt.-% of fatty acids selected from the group of fatty acids having 7, 9 and 11 carbon atoms. Preferred is tripheptanoin.

The amount of the triglyceride b), preferably the mid-chain triglycerides in the emulsion of the present invention may range from 2 to 40, preferably 5 to 25, and most preferably from 8 to 18 weight-% (wt.-%), based on the total weight of the emulsion.

In a preferred aspect of the invention the amount of the semifluorinated compound a) and the triglyceride b), which is preferably the mid-chain triglyceride ranges from 5 to 40 wt.-%, preferably 10 to 30 wt.-%, especially 15 to 25 wt.-%, in particular 18 to 22 wt.-%, e.g. about 20 wt.-%, based on the total weight of the emulsion.

A good balance of a high concentration of the semifluorinated compound and an excellent long term stability can be achieved, if the weight ratio of the semifluorinated carbon compound a) to the triglyceride, which is preferably the mid-chain triglyceride (MCT) ranges from 1:20 to 1:0.7, preferably 1:15 to 1:0.8, more preferably 1:10 to 1:0.9, more preferably 1:4 to 1:1, especially 1:2 to 1:1,e.g. about 1:1.5.

In an alternative embodiment the weight ratio of component a) to component b) is from 1:20 to 1:0.20, preferably 1:15 to 1:0.25, more preferably 1:10 to 1:0.30, further preferably 1:4 to 1:0.35, especially 1:2 to 1:0.4.

The emulsion of the invention comprises the semifluorinated compound and preferably the mid-chain triglyceride (MCT). According to one aspect of the invention the oil components of the emulsion form a homogeneous solution at 20 °C. MCT and the semifluorinated compound such as the F6H8 are miscible at 20 °C and thus form a homogeneous solution. The oil phase may comprise further oil components such as long chain fatty acid triglycerides based on vegetable oil or marine oils such as fish oil. The amount of the further oil components is preferable adjusted that a completely miscible oil phase is maintained. Generally, the amount of oil components which are different from the semifluorinated compound and triglyceride b) which is preferably the MCT, are less than 50 weight percent (wt.-%), preferably less than 40 wt.-%, more preferably less than 30 wt.-%, further preferably less than 20 wt.-%, especially less than 10 wt.-%, in particular less than 5 wt.-% or essentially free, wherein the amount is based on the total weight of the oil components.

The weight ratio of MCT to long chain triglycerides (LCT) in the emulsion of the invention may generally range from 1:1 to 10000:1 or more, preferably ranging from 3:2 to 1000:1, more preferably ranging from 3:1 to 500:1, especially 5:1 to 200:1.

Oils which may be present in the emulsions of the invention and which are different from MCT may be selected from "long-chain triglycerides" (LCT).

In certain embodiments the oil may comprise a vegetable oil. "Vegetable oil" refers to oil derived from plant seeds or nuts. Vegetable oils are typically "long-chain triglycerides" (LCTs), formed when three fatty acids (usually 14 to 22 carbons in length, with unsaturated bonds in varying numbers and locations, depending on the source of the oil) form ester bonds with the three hydroxyl groups on glycerol. In certain embodiments, vegetable oils of highly purified grade (also called "super refined") are used to ensure safety and stability of the oil-in-water emulsions. In certain embodiments hydrogenated vegetable oils, which are produced by controlled hydrogenation of the vegetable oil, may be used.

Exemplary vegetable oils include but are not limited to almond oil, babassu oil, black currant seed oil, borage oil, canola oil, caster oil, coconut oil, corn oil, cottonseed oil, olive oil, peanut oil, palm oil, palm kernel oil, rapeseed oil, safflower oil, soybean oil, sunflower oil and sesame oil. Hydrogenated and/or or partially hydrogenated forms of these oils may also be used. In specific embodiments, the oil additionally comprises safflower oil, sesame oil, corn oil, olive oil and/or soybean oil. In more specific embodiments, the oil additionally comprises safflower oil, and/or soybean oil.

In specific embodiments where the oil additionally comprises soy bean oil, the soybean oil may have a palmitic acid content (wt./wt) of between 9 and 13%, a stearic acid content of between 2.5% and 5%, an oleic acid content of between 17% and 30%, a linoleic acid content of between 48% and 58%, and a linolenic acid content of between 5% and 11%.

Further, in a specific embodiment, the aqueous emulsion of the invention may comprise structured triglycerides. A "structured triglyceride" as used herein is a triglyceride comprising triglycerides or mixtures of triglycerides having at least one fatty acid group with a carbon chain length of from 6 to 12 carbon atoms and at least one fatty acid group with a carbon chain length of more than 12 carbon units.

A further component of the emulsion of the invention is an emulsifier. Preferably, the emulsifier is selected from the group consisting of phospholipids, amphoteric and ionic surfactants such as fatty acid salts.

The emulsifier may be present in the emulsion in an amount up to 10 wt.-%, preferably 0.5 to 5 wt.-% based on the total weight of the emulsion.

Preferably, the emulsifier is a phospholipid or a mixture of phospholipids.

In one aspect the emulsifier may comprise lecithins, preferably naturally occurring lecithins such as soy lecithin, egg lecithin, sunflower oil lecithin, sphingosine, gangliosides, phytosphingosine, and combinations thereof. Hydrogenated lecithin, i.e. the product of controlled hydrogenation of lecithin, may also be additionally used in the emulsifier.

Exemplary phospholipids useful in the present invention include, but are not limited to phosphatidyl choline, phosphatidylethanolamine, phosphatidylglycerol, phosphatidic acid, lyso-phosphtidylcholine and mixtures thereof. The phospholipid component of the compositions can be either a single phospholipid or a mixture of several phospholipids. The phospholipids employed may be natural or synthetic, but should be acceptable for parenteral, especially intravenous, administration.

A non-exhaustive list of suitable phospholipids which may additionally present in the emulsifier is listed below:
Phosphatidic acids, including 1,2-Dimyristoyl-sn-glycero-3-phosphatidic acid, sodium salt (DMPA,Na), 1,2-Dipalmitoyl-sn-glycero-3-phosphatidic acid, sodium salt (DPPA,Na), 1,2-Distearoyl-sn-glycero-3-phosphatidic acid, sodium salt (DSPA,Na); phosphocholines, including 1,2-Dilauroyl-sn-glycero-3-phosphocholine (DLPC), 1,2-Dimyristoyl-sn-glycero-3-phosphocholine (DMPC), 1,2-Dipalmitoyl-sn-glycero-3-phosphocholine (DPPC), 1,2-Distearoyl-sn-glycero-3-phosphocholine (DSPC); phosphoethanolamines, including 1,2-Dilauroyl-sn-glycero-3-phosphoethanolamine (DLPE), 1,2-Dimyristoyl-sn-glycero-3-phosphoethanolamine (DMPE), 1,2-Dipalmitoyl-sn-glycero-3-phosphoethanolamine (DPPE), 1,2-Distearoyl-sn-glycero-3-phosphoethanolamine (DSPE); phosphoglycerols, including 1,2-Dilauroyl-sn-glycero-3-phosphoglycerol, sodium salt (DLPG, Na), 1,2-Dimyristoyl-sn-glycero-3-phosphoglycerol, sodium salt (DMPG, Na), 1,2-Dimyristoyl-sn-glycero-3-phospho-sn-1-glycerol, ammonium salt (DMP-sn-1-G,NH4), 1,2-Dipalmitoyl-sn-glycero-3-phosphoglycerol, sodium salt (DPPG,Na), 1,2-Distearoyl-sn-glycero-3-phosphoglycerol, sodium salt (DSPG,Na), 1,2-Distearoyl-sn-glycero-3-phospho-sn-1-glycerol, sodium salt (DSP-sn-1G,Na); phosphoserines, including 1,2-Dipalmitoyl-sn-glycero-3-phospho-L-serine, sodium salt (DPPS,Na); mixed chain phospholipids, including 1-Palmitoyl-2-oleoyl-sn-glycero-3-phosphocholine (POPC), 1-Palmitoyl-2-oleoyl-sn-glycero-3-phosphoglycerol, sodium salt (POPG,Na), 1-Palmitoyl-2-oleoyl-sn-glycero-3-phosphoglycerol, ammonium salt (POPG,NH4); lysophospholipids, including 1-Palmitoyl-2-lyso-sn-glycero-3-phosphocholine (P-lyso-PC), 1-Stearoyl-2-lyso-sn-glycero-3-phosphocholine (S-lyso-PC); pegylated phospholipids, including N-(Carbonyl-methoxypolyethyleneglycol 2000)-MPEG-2000-DPPE, sodium salt, N-(Carbonyl-methoxypolyethyleneglycol 5000)-MPEG-5000-DSPE, sodium salt, N-(Carbonyl-methoxypolyethyleneglycol 5000)-MPEG-5000-DPPE, sodium salt, N-(Carbonyl-methoxypolyethyleneglycol 750)-MPEG-750-DSPE, sodium salt, N-(Carbonyl-methoxypolyethyleneglycol 2000)-MPEG-2000-DSPE, sodium salt.

In one embodiment the amount of phospholipid in the emulsion of the present invention, by weight based on the total weight of the composition, is within a range of 0.1 to 30 wt.-%. In certain embodiments, phospholipid may be present within a range of 0.5 to 15 wt.-%, including 0.8 to 10 wt.-%, such as 1 to 5 wt.-%.

In an exemplary embodiment the amount of phospholipid in the aqueous emulsion of the present invention is up to 10 weight% (wt.-%), preferably 0.5 to 5 wt.-% based on the total weight of the emulsion.

In one embodiment, the emulsifier comprises egg lecithin comprising 60-80% wt/wt, such as 67% wt/wt phospatidyl choline; 10-20% wt/wt, such as 15% wt./wt, phospatidlylethanolamine; <=3% wt/wt, such as 2% wt./wt, sphingomyelin; and <=3% wt/wt, such as 1% wt/wt, lysophosphatidylcholine.

"Egg lecithin PL90" (Fresenius Kabi AB) is one example of an egg lecithin having such a phospholipid content.

In particular good results can be achieved with an emulsifier comprising a lecithin comprising about 80 to about 85 wt.-% phosphatidylcholine;
about 7.0 to about 9.5 wt.-% phsphatidylethanolamine;
less than 3 wt.-% lysophosphatidylcholine;
less than 0.5 wt.-% lysophosphatidylethanolamine; and
about 2 to about 3 wt.-% sphingomyelin.

According to a preferred embodiment of the present invention the emulsifier comprises a mixture of phospholipids and glycolipids. It has surprisingly been found that mixtures of phospholipids and glycolipids further improve the storage stability of the emulsions at higher temperatures (40 °C). In particular these mixtures stabilize the emulsions even under stress conditions like the sterilization of the emulsions. The emulsions of the invention are usually sterilized, for example at 121°C for 15 min at a pressure of 2 bar in a rotational autoclave.

Glycolipids are lipids with a carbohydrate attached. Glycolipids are phosphorus free membrane lipids of cell membranes wherein one or more mono or oligosaccharides are connected to a lipid. The lipids are fatty acids which are connected via ester bonds with glycerol or via amide bonds with sphingosin.

In a preferred embodiment the emulsion of the invention additionally comprises glycolipids, which are preferably selected from glyceroglycolipids, such as mono galactosyldiglyceride or glycosphingolipids or cerebrosides.

In a preferred embodiment the amount of glycolipids is 5 to 30 wt.-%, preferably 10 to 28 wt.-% based on the total weight of the sum of glycolipid and phospholipid. Mixtures of phospholipids and glycolipids are commercially available as Lipoid S 75 ex Lipoid GmbH, Germany.

In a special preferred embodiment the emulsifier comprises
about 68 to about 73 wt.-% phosphatidylcholine;
about 7 to 10 wt.-% phosphatidylethanolamine;
less than about 3 wt.-% lysophosphatidylcholine; and
about 14 to about 25 wt.-% glycolipides, wherein all weight are based on the total amount of sum of glycolipids and phospholipids.

The emulsion preferably comprises 0.5 to 5 wt.-%, more preferably 1.0 to 4.0 wt.-% of an emulsifier wherein the amount is based on the total weight of the emulsion.

The emulsion of the invention is preferably an oil in water emulsion wherein the aqueous phase is the continuous phase and the oily phase is the discontinuous phase.

As noted above, the oil-in-water emulsion of the present invention further comprises an aqueous medium. "Aqueous medium" or "aqueous phase" refers to a water-containing liquid. In some embodiments, the aqueous medium is water and/or an aqueous buffer solution.

The aqueous emulsion, preferably the oil-in water emulsion, of the invention may comprise 70 to 98 wt.-%, preferably 70 to 90 wt.-% water, based on the total weight of the emulsion.

In some embodiments, the emulsion may comprise 0 to 4 mM, preferably 0.5 to 3 mM of a physiologically compatible buffering agent.

In some embodiments, the aqueous emulsions according to the present invention optionally comprise a co-surfactant. Co-surfactants suitable for use in the emulsions of the present invention are those that prevent flocculation and/or coalescence of the lipid emulsion. Exemplary co-surfactants include, but are not limited to cholesterol, oleic acid, oleate, Tween80 (PEG-sorbitan monooleate), HCO-60, Solutol H15 (polyoxyethylene-660-hydroxystearate), PEG-400 (polyethylene glycol), Pluronic F68 (BASF), Cremophor EL (polyoxyethylene-35-ricinoleate), or the salt of a bile acid, such as deoxycholic acid. In other embodiments the co-surfactant is selected from the group consisting of C12-C22 fatty acids, salts thereof, and/or mixtures thereof, such as from C16-C20 fatty acids, salts thereof, and/or mixtures thereof, or from C18 fatty acids, salts thereof, and/or mixtures thereof. In specific embodiments, the fatty acid is mono-unsaturated.

In some embodiments the co-surfactant may be present in the emulsion in an amount (wt.-%) greater than or equal to 0.005%, greater than or equal to 0.01%, or greater than or equal to 0.02%. In accordance with any of these embodiments the co-surfactant may be present in an amount (wt.-%) less than or equal to 4%, less than or equal to 1%, or less than or equal to 0.04%.
In specific embodiments, the co-surfactant is selected from the group consisting of long-chain fatty acids, such as palmitic acid, oleic acid or stearic acid, or the alkali salts thereof. Oleate and/or oleic acid, particularly sodium oleate, are particularly suitable co-surfactants.

In certain embodiments where the co-surfactant is oleate and/or oleic acid, the co-surfactant may be present in an amount (wt.-%) equal to or greater than 0.005 wt.-%, equal to or greater than 0.01 wt.-%, or equal to or greater than 0.02 wt.-%. In accordance with any of these embodiments, the co-surfactant may be present in an amount (wt.-%) less than or equal to 0.5 wt.-%, less than or equal to 0.2 wt.-%, less than or equal to 0.1 wt.-%, or less than or equal to 0.05 wt.-%. In specific embodiments, the co-surfactant is sodium oleate and is present in an amount of 0.03 wt.-% (0.3 g/l).The emulsions described herein may be suitable for parenteral infusion, such as intravenous infusion. In specific embodiments, the concentration of certain co-surfactants therefore is kept to a minimum to prevent side effects such as irritation, cytochrome P450 inhibition, etc. In specific embodiments, Pluronic F68 (poly(ethyleneglycol)-13-poly(propylene glycol co-propylene glycol) is present in an amount less than 0.7 wt.-%, or less than 0.5 wt.-%. In other specific embodiments, Solutol-HS (Macrogol-15-hydroxystearate) is present in an amount less than 1.2 wt.-%, or less than 1 wt.-%.

The oil-in water emulsion according to the invention may comprise a tonicity agent. Such compositions may have an osmolality in the range of 200-1000 mOsm/kg, preferably 220 to 800 mOsm/kg, especially 250 to 600 mOsm/kg.

In accordance with specific embodiments of the invention the emulsions may be isotonic and iso-osmotic. The compositions may have an osmolality of 220-600 mOsm/kg, or 230-360 mOsm/kg.

Suitable tonicity agents include potassium or sodium chloride, trahalose, sucrose, sorbitol, glycerol, glucose, xylitol, mannitol, polyethylene glycol, propylene glycol, albumin, amino acid and mixtures thereof. In certain embodiments, an osmolality of 270 to 330 mOsm/kg, such as 280 to 300 mOsm/kg, is achieved with an agent that also increases osmotic pressure, such as glycerol, dextrose, lactose, sorbitol or sucrose.

In one embodiment, the tonicity agent is a physiologically acceptable polyol, such as glycerol, sorbitol or xylitol. In a specific embodiment, the tonicity agent is glycerol.

Thus, according to a further aspect of the invention the emulsion additionally comprises a tonicity agent, preferably glycerol.

The tonicity agent may be present in an amount ranging from 0.1 to 10 wt.-%, preferably 0.5 to 8 wt.-%, more preferably 1 to 5 wt.-%, based on the total weight of the emulsion.

The tonicity agent is generally used in an amount that does not have adverse biological effects, but is sufficient to provide isosmotic and/or isotonic compositions. When glycerol is the tonicity agent, glycerol may be present in the range of 2 to 5% (wt.-%), such as 2.1% to 2.9% (wt.-%), including 2.3% to 2.7%. In specific embodiments, the emulsions of the present invention comprise 2.5% glycerol (25 g/l). In a further embodiment the tonicity agent is present in an amount of 2.5 wt.-% or more, based on the total weight of the emulsion.

In some embodiments, the emulsions according to the present invention have a pH within the range of pH 6.0 to pH 9.0, such as pH 6.5 to pH 8.5, including pH 7.5 to 8.5, such as 8.0 to 8.5. In an alternative embodiment the pH can range within 5.0 to 9.0. The pH of the compositions may be adjusted by methods known in the art, e.g., through the use of an appropriate base that neutralizes the negative charge on the fatty acids, through the use of an appropriate buffer, or a combination thereof. A variety of bases and buffers are suitable for use with the emulsions of the present invention. One skilled in the art will appreciate that the addition of buffer to the emulsion will affect not only the final pH, but also the ionic strength of the emulsion. High ionic strength buffers may negatively impact the zeta potential of the emulsion and are, therefore, not desirable. In a specific embodiment, the pH is adjusted to the desired value by addition of sodium hydroxide, such as a 0.1 N sodium hydroxide or 1 N sodium hydroxide. In order to further stabilize the emulsion of the invention against oxidative processes an antioxidant may be present. In a preferred embodiment the emulsion additionally comprises an antioxidant, preferably alpha-tocopherol.

The emulsion according to the present invention optionally comprise one or more pharmaceutically acceptable additives, such as acidifying, alkalizing, binding, chelating, complexing, solubilising agents, antiseptics, preservatives (including antimicrobials and antioxidants), suspending agents, stabilizing agents, wetting agents, viscosity modifying agents, solvents, cryo-protectants, diluents, lubricants and other biocompatible materials or therapeutic agents. In certain embodiments, such additives assist in stabilizing the further the emulsion or in rendering the emulsions of the present invention biocompatible.

According to a preferred embodiment of the invention the density at 25 °C of the oil phase, in particular the density of the mixture consisting of triglyceride b) which is preferably MCT, and semifluorinated compound, ranges between 0.9 to 1.1 g/cm³, more preferably ranges between 0.95 and 1.05 g/cm³ or about 0.95 to about 1.10 g/cm³. In a further aspect of the invention the density of the oil phase does not deviate more than 10 %, preferably not more than 5 %, especially not more than 3 % from the density of the aqueous phase. The aqueous phase comprises all ingredients which are in the continuous phase and the oil phase comprises all components which are in the discontinuous phase.

It has been found that good diagnostic results can be achieved with emulsions having an oil droplet size in the nano range. According to a preferred aspect of the invention the particles of the discontinuous phase have an average particle diameter preferably ranging from 1 to 500 nm, more preferably from 10 to 400 nm and further preferably from 50 to 350 nm, measured with photon correlation spectroscopy (PCS) at 25 °C.

A further embodiment of the invention is the emulsion of the invention for use as a medicament.

It has been found that the semifluorinated compound present in the emulsion of the present invention is taken up by monocytes/macrophages in such a way that the cells become specifically labeled, which can be utilized for diagnosis in imaging methods. Surprisingly, a diagnostic potential for the visualization of inflammatory processes, in particular of the cardiovascular system, and lymph nodes was found.

Therefore, the emulsions of the inventions are suitable to be used as a contrast agent or contrast enhancement agent. In particular for the nuclear magnetic resonance imaging techniques the emulsions are suitable since said semifluorinated carbon compound of the emulsions are specifically enriched and distributed in the inflammatory tissues.

Another aspect of the invention is the emulsion of the invention for use as a contrast enhancement agent or contrast agent for the diagnostic detection, by means of an imaging procedure utilizing MR-techniques.

A further embodiment of the present invention is the emulsion of the invention for use as a constrast agent for the diagnostic detection, by means of an imaging procedure, in particular where the imaging process is based on measuring the nuclear magnetic resonance of the ¹⁹F isotope.

The evaluation of the corresponding measurements and conversion thereof into an image is known to a skilled person, as can be seen, for example, from:
Haacke M E, Brown W R, Thompson M R, Venkasetan R: Magnetic Resonance Imaging-Physical Principles and Sequence Design, Wiley, New York, 1999;
Yu J X, Kodibagkar V D, Cui W, Mason R P. 19F: a versatile reporter for non-invasive physiology and pharmacology using magnetic resonance; Curr Med Chem. 12: 819-48, 2005;
Wernick M N, Aarsvold J N Emission Tomography: The Fundamentals of PET and SPECT, Academic Press, London, 2004.

The emulsions are in particular suitable to detect inflammatory processes. According to a further aspect of the invention the emulsion is for the diagnostic detection, by means of an imaging procedure, of inflammatory processes selected from the group consisting of inflammatory reaction peripheral to infarctions such as myocardial infarction, stroke; inflammation of organs, such as myocarditis, encephalitis, meningitis; multiple sclerosis; inflammation of the gastrointestinal tract, such as Crohn's disease; inflammation of the vessels, such as arteriosclerosis, in particular vulnerable plaques; detection of abscesses and also arthritis, wherein the imaging process is based on measuring the nuclear magnetic resonance of the ¹⁹F isotope.

In another aspect of the invention the emulsion is for use in the diagnostic detection, based on non-invasive imaging procedures of the cardio-vascular system, including the myocardium, arteries and veins; inflammatory reactions occurring in disease processes like myocardial infarction, myocarditis, atherosclerosis and thrombosis leading to inflammatory and degenerative processes of the vasculature found in neurology such as stroke or tumor; pulmology such as thrombosis, inflammation, sarcoidosis; gastroenterology such as tumour, inflammatory bowl diseases such as Crohn's disease; and rheumatology such as autoimmune diseases of the vessels such as Takayasu arteritis.

A further embodiment of the invention refers to a method comprising acquiring non-invasive imaging procedures of a patient to whom the emulsion of the invention has previously administered so as to dissolve it in the patient's bloodstream.

Preferably, the non-invasive imaging procedure is a MR imaging procedure.

In a further preferred aspect of the method of the invention the imaging process is based on measuring the nuclear magnetic resonance of the ¹⁹F isotope or the ¹⁹F isotope and the ¹H isotope.

The presence of ¹⁹F and/or ¹⁸F isotopes in the fluorinated compounds allows the advantage of using devices already known and existent in the hospital, namely magnetic nuclear resonance spectroscopy with ¹⁹F isotopes and/or the use of positron emission spectroscopy of ¹⁸F isotopes.

By the use of the emulsion according to the invention the following pathological conditions can especially be detected:
1) Visualization of lymph nodes and their pathological enlargement
   a) cancers that directly affect the lymph nodes: Hodgkin's disease, non-Hodgkin lymphomas;
   b) tumor metastases, for example, from breast cancer;
   c) viral and bacterial infections, for example, syphilis, tuberculosis;
2) Inflammation reactions in the border zone of
   a) infarctions, for example, myocardial infarction, stroke;
   b) tumors;
3) inflammation of organs: myocarditis, encephalitis, meningitis (cerebral and spinal meninges);
4) multiple sclerosis;
5) inflammations of the gastrointestinal tract, for example, Crohn's disease;
6) inflammation of the vessels, for example, arteriosclerosis, especially so-called "vulnerable plaques";
7) detection of abscesses;
8) detection of arthritis.

The emulsion of the invention is usually administered parenterally prior to start the imaging process. In a preferred embodiment the emulsion is administered intravenously or intraarticularly.

A further aspect of the invention is a method for the detection of an inflammatory process comprising:
(a) administering the emulsion of the invention to a subject in need thereof; and
(b) imaging the fluorine-containing compound in said subject,
   wherein said inflammatory process is preferably selected from the group consisting of:
   a cancer that directly affects the lymph nodes; an inflammatory process in the border zone of an infarction or tumor; inflammation of an organ; multiple sclerosis; inflammation of the gastrointestinal tract; inflammation of the vessels; arthritis; and abscess.

A further embodiment of the invention is a method for the manufacturing of the emulsion of the invention comprising the following steps:
i) providing a mixture comprising
   a) a semifluorinated compound of formula I:

      CF₃-(CF₂)ₓ-(CH₂)_{y}-CH₃ (I)

      wherein x is an integer ranging from 1 to 8 and y is an integer ranging from 1 to 10,
   b) a triglyceride which is miscible with the semifluorinated compound at 20°C, preferably a mid-chain triglyceride (MCT) and
   c) an emulsifier, and
   d) water; and
ii) homogenizing the mixture, preferably homogenizing the mixture in a high pressure homogenizer;
iii) optionally sterilizing the emulsion.

In another aspect the manufacturing method comprises the steps:
i) dissolving the semifluorinated compound in triglyceride b) which is preferably the mid-chain triglyceride; and
ii) emulsifying the oil phase in the aqueous phase, preferably in the presence of the emulsifier.

According to a specific embodiment, the aqueous composition is homogenized so as to produce a homogeneous suspension, before said aqueous composition is combined with the oily composition.

In a specific embodiment, the homogenization is performed at greater than or equal to 350 bar, or greater than or equal to 370 bar.

In specific embodiments, the methods of manufacturing of the emulsions involve the steps of dissolving emulsifier in aqueous medium (rather than in oil), adding the oil phase to the aqueous phase (rather than vice versa), and homogenization at greater than or equal to 350 bar. These steps result in emulsions with advantageous properties in terms of particle size and emulsion stability.

In another specific embodiment, the emulsion is packaged in sealed containers, and sterilized, such as by heating to at least 121°C (e.g. 121°C to 123°C) for a minimum of 15 mins holding time. The autoclave program may be a rotary cycle.

A further embodiment of the invention is a container containing the emulsion of the invention. The material of the container is preferably selected from the group consisting of glass and organic polymers and mixtures thereof. Preferred organic polymers are polyethylene and/or polypropylene.

In a preferred embodiment of the manufacturing method of the invention said method comprises the following steps:
a) preparing a mixture by
   a-1) preparing a mixture comprising water and an emulsifier,
   a-2) preparing a homogenous mixture comprising the semifluorinated compound and the triglyceride,
   a-3) combining the mixture obtained in step a-1) with the mixture obtained in step a-2); and
b) homogenizing the mixture obtained in step a), preferably homogenizing the mixture obtained in step a) with a high pressure homogenizer.

In an especially preferred embodiment of the manufacturing method of the emulsion of the present invention said method comprises the following steps:
a) preparing a mixture by
   a-1) preparing a mixture comprising the water soluble components of the emulsion, preferably a mixture comprising water, emulsifier and tonicity agent, in particular a mixture comprising water, phospholipid, sodium oleate and glycerol,
   a-2) preparing a homogeneous mixture of the oil soluble components of the emulsion, preferably a mixture comprising the semifluorinated compound, the triglyceride and an antioxidant, more preferably a mixture comprising F6H8, MCT and alpha-tocopherol,
   a-3) combining the mixture obtained in step a-1) with a mixture obtained in step a-2); and
b) homogenizing the mixture obtained in step a) to prepare a pre-emulsion;
c) further homogenizing the mixture obtained in step b) by a high pressure homogenizer.

It has surprisingly been found that best results can be obtained by homogenizing the emulsion in step b) and step c) in a preferred temperature range between 30 to 70 °C, more preferably between 40 and 60 °C.

In a further preferred embodiment of the manufacturing method a step d) is applied wherein the emulsion is sterilized, preferably by heating to at least 121 °C for a minimum of 15 minutes.

The preferred amounts and components of the emulsion are mentioned in conjunction with emulsion of the invention.

A further aspect of the invention is a method for operating a magnetic resonance tomograph. In this method an alternating magnetic field is emitted and an absorption of the emitted magnetic field by a sample to be examined is detected. These method steps correspond to the usual operation of a magnetic resonance tomograph. The inventive method serves for the detection of a semifluorinated compound of formula I:

CF₃-(CF₂)ₓ-(CH₂)_{y}-CH₃ (I)

wherein x is an integer ranging from 1 to 8 and y is an integer ranging from 2 to 10 in an emulsion of the present invention.

The inventive method is characterized in that the frequency of the alternating magnetic field is between B x 40.045600 MHz/T and B x 40.065600 MHz/T, B being the magnetic field strength in Tesla.

In order to obtain the above-mentioned frequency for the alternating magnetic field the following method steps have been performed:
First, the frequency of the alternating magnetic field has been chosen with B x 40.05560 MHz/T. In order to detect an unknown compound containing a fluoro atom a broad excitation and receiving band width (e.g. +/- 5 kHz) is used. In the middle this excitation band is the excitation frequency of the fluoro atom core (40.05560 MHz/T). This frequency is defined as the zero position.

During excitation the characteristic spectrum of the unknown compound containing fluorine appears at a certain offset frequency. If this is not the case the chosen band width (e.g. +/- 5 kHz) has to be widened.

After the specific spectrum has been found at a certain frequency the excitation spectrum is offset by choosing an appropriate offset frequency. The inventors have found that an offset frequency of +/-100 Hz to +/- 10000 Hz is appropriate. By choosing such a frequency the excitation and receiving band width can be significantly reduced, thus obtaining a reduction of noise so that a better signal to noise ratio can be obtained.

A further aspect of the invention relates to a method for detecting in a sample to be examined a semifluorinated compound of formula I:

CF₃-(CF₂)ₓ-(CH₂)_{y}-CH₃ (I)

wherein x is an integer ranging from 1 to 8 and y is an integer ranging from 2 to 10 in an emulsion of the invention. In this method an alternating magnetic field is emitted and an absorption of the emitted magnetic field by a sample to be examined is detected. These steps correspond to the usual operation of a magnetic resonance tomograph.

According to this invention at least one ¹⁹F fluorine proton image is super imposed over a ¹H proton image. Hereby the protons of ¹H image is displayed as a grey scale image and the protons of ¹⁹F fluorine image is displayed by using a color gradient, one end of this color gradient indicating a low concentration of ¹⁹F fluorine protons and the other end of this color gradient indicating a high concentration of ¹⁹F fluorine protons.

These method steps can be applied for example for assigning the detected fluorine signal to a certain part of a patient's body. For this purpose it is possible to interpolate the ¹⁹F-volume and the ¹H-volume in order to obtain a better resolution. As explained the ¹H proton image is displayed as a grey scale image whereas the ¹⁹F fluorine proton image can be displayed for example as a color gradient changing from black to green or from blue to red for example.

First, a three-dimensional video is generated. However, it is preferred that subsequently two-dimensional images of each layer are generated, each of them showing a cross section of the sample to be examined. It is preferred that maximum intensity projections (MIP's) are calculated. These two-dimensional layer images show in a comprehensive manner in which anatomic structure the contrast agent is present.

In a preferred embodiment of this invention disturbing signals generated by ¹⁹F fluorine protons in a second sample not to be examined are eliminated. Such disturbing signals can for example be generated by contrast agent in the liver or spleen of a patient. Contrast agent in the liver can cause a very strong disturbing signal when during an imaging process it is tried to detect for example an inflammation in the heart region of a patient, since the liver is located very close to the heart.

In order to eliminate such disturbing signals it is possible to compare the value for the fluorine concentration in a first pixel with the values of all neighboring pixels in the three-dimensional space around this first pixel. Hereby, if the difference between the values for the fluorine concentration of two adjacent pixels is below a threshold, these two pixels are considered to belong to a cluster of pixels, e.g. to the liver of the patient. This disturbing cluster of pixels can be removed from the image obtained if this cluster does not represent the sample to be examined (in this case the heart of the patient). Removal of this disturbing cluster can be performed for example manually.

When superimposing ¹⁹F fluorine images with the ¹H proton image it is preferred that these images have been obtained without changing the measuring geometry which means that both data sets contain signals from the same volume.

Noise which may be present in the ¹⁹F fluorine proton images can be suppressed by averaging the different acquisitions. The standard deviation σ of the noise is measured whereby 3 ▪ σ or 5 ▪ σ can be used as a threshold for the signal to be detected. This means that only signals which are three times higher than the standard deviation of the noise are considered to be a "real" signal. This threshold can be adapted by the user so that it is possible to adapt the images obtained to the amount of noise. It is preferred that the last mentioned method steps are not applied to the ¹H proton images since these have a higher SNR.

All the method steps which have been discussed in the context of the last mentioned invention are basically related to signal processing steps and therefore can be performed independently from the data acquisition process at any time and any place. In particular, performing these method steps does not require the presence of a patient's body.

Preferred embodiments of the emulsions used in the methods of the invention are described in conjunction with the emulsion of the invention.

In the following, the invention is further illustrated by way of examples:

### Experimental section

### Examples 1-3

The emulsions of the experimental section have generally been prepared as follows:
1) mixing the emulsifier with water;
2) preparing a mixture of the hydrophobic components such as MCT, fluorinated compound and optionally α-tocopherol;
3) adding the aqueous mixture from step 1) to the oil mixture of step 2);
4) homogenizing the emulsion with a high pressure homogenizer 3 times at 700 bar and 2 times at 1000 bar in a Panda^{®} Plus 2000 high pressure homogenizer.

Table 1 shows oil in water emulsion compositions and Table 2 shows the respective storage stability data at 20°C. The amounts referred to in the Table are weight-% (wt.-%) based on the total weight of the emulsion.

**Table 1: Oil in water emulsion compositions according to examples 1 to 3 of the invention and Comparative Example 9**

| Component | Example 1 | Example 2 | Example 3 | Comparative Example 9 |
|---|---|---|---|---|
| F6H8 ¹⁾ | 4 | 6 | 8 | 20 |
| MCT²⁾ | 16 | 14 | 12 | - |
| Lipoid E 80 ³⁾ | 2 | 2 | 2 | 2 |
| Water | Ad 100 | Ad 100 | Ad 100 | Ad 100 |

| | | | | |
|---|---|---|---|---|
| ¹⁾ Perfluorhexyloctane ex Novaliq, Germany ²⁾ Mid chain triglycerides (50 - 80 wt.-% C₈-fatty acid and 20 - 50 wt.-% C₁₀-fatty acid) ³⁾ Egg phospholipid ex Lipoid GmbH, Germany | | | | |

**Table 2: Storage stability data of the emulsions according to examples 1 to 3**

| Example | Storage time | ZAverage | P.I. | pH |
|---|---|---|---|---|
| | [days] | [nm] | | |
| 1 | 0 | 148,3 | 0,073 | 8,09 |
| | 7 | 158,1 | 0,093 | 6,61 |
| | 14 | 169,2 | 0,098 | 6,12 |
| | 28 | 173,9 | 0,098 | 5,83 |
| 2 | 0 | 166,1 | 0,054 | 8,10 |
| | 7 | 173,6 | 0,075 | 6,59 |
| | 14 | 184,2 | 0,061 | 6,19 |
| | 28 | 185,0 | 0,069 | 5,96 |
| 3 | 0 | 155,7 | 0,075 | 8,11 |
| | 7 | 167,7 | 0,065 | 6,62 |
| | 14 | 176,7 | 0,068 | 6,08 |
| | 28 | 178,5 | 0,090 | 5,98 |

Average particle diameter (ZAverage) and the polydispersity index (P.I.) have been determined by photon correlation spectroscopy (PCS).

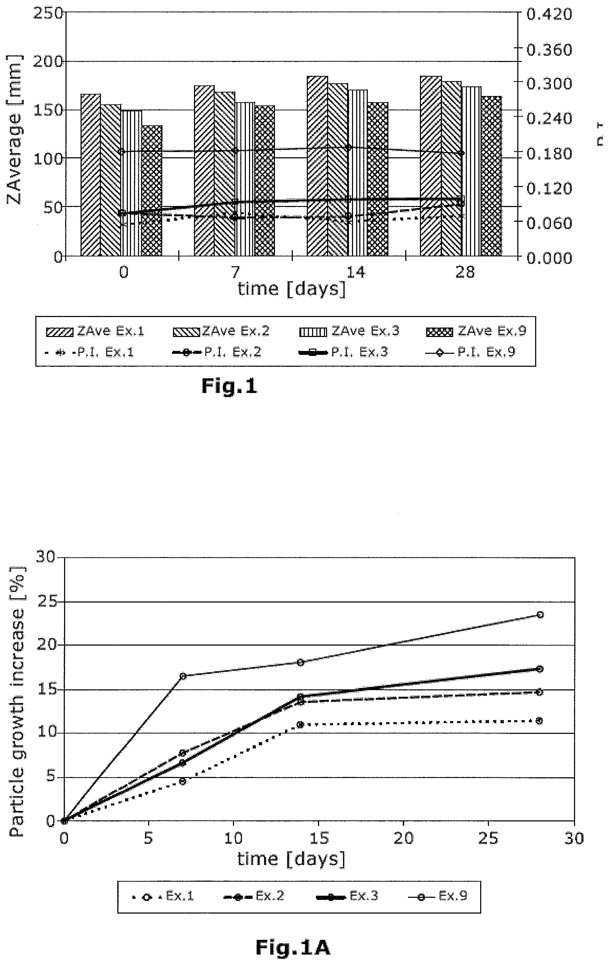

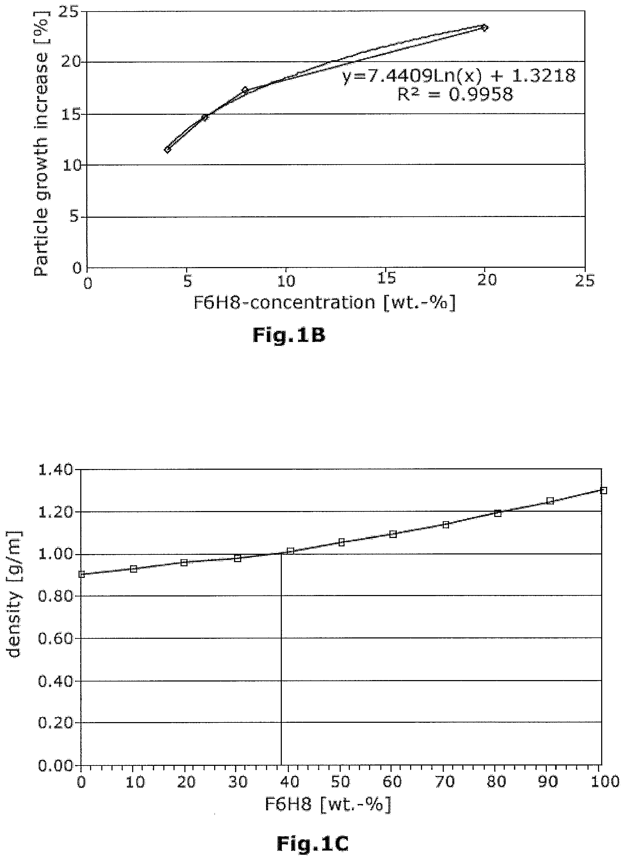

Figure 1 shows the data for the storage stability tests as reflected in Table 2 as well as the data concerning the comparative example 9.
Figure 1A shows the particle growth of the emulsions according to examples 1 to 3 as well as comparative example 9. The particle growth measurement started immediately after the manufacturing of the emulsion.
Figure 1A shows that the average particle diameter of the oil droplets within 28 days increases by about 11 % for example 1, by about 15 % for example 2, by about 17 % for example 3 and by about 23 % for comparative example 9. The particle growth increase is higher the higher the concentration of the semifluorinated compound is.
Fig. 1B shows the dependency of the particle growth increase on the concentration of the semifluorinated compound F6H8.

Table 2A reflects the data which are shown in Figures 1, 1A and 1B. The storage tests have been carried out at 20 °C.

**Table 2A**

| Example | Storage time | ZAverage | P.I. | Absolute Particle growth increase | Relative Particle growth increase |
|---|---|---|---|---|---|
| | [days] | [nm] | | [nm] | [%] |
| 1 | 0 | 148,3 | 0,073 | 0,0 | 0,0 |
| | 7 | 158,1 | 0,093 | 7,5 | 4,5 |
| | 14 | 169,2 | 0,098 | 18,1 | 10,9 |
| | 28 | 173,9 | 0,098 | 18,9 | 11,4 |
| 2 | 0 | 166,1 | 0,054 | 0,0 | 0,0 |
| | 7 | 173,6 | 0,075 | 12,0 | 7,7 |
| | 14 | 184,2 | 0,061 | 21,0 | 13,5 |
| | 28 | 185,0 | 0,069 | 22,8 | 14,6 |
| 3 | 0 | 155,7 | 0,075 | 0,0 | 0,0 |
| | 7 | 167,7 | 0,065 | 9,8 | 6,6 |
| | 14 | 176,7 | 0,068 | 10,9 | 14,1 |
| | 28 | 178,5 | 0,090 | 25,6 | 17,3 |
| Comparative Example 9 | 0 | 132,4 | 0,181 | 0,0 | 0,0 |
| | 7 | 154,1 | 0,183 | 21,7 | 16,4 |
| | 14 | 156,2 | 0,187 | 23,8 | 18,0 |
| | 28 | 163,4 | 0,177 | 31,0 | 23,4 |

It has been found that an optimized balance of a high concentration of the semifluorinated compound (which is necessary to achieve a good contrast in the MR measurement) and a storage stable emulsion can be achieved if the density of the oil droplets (MCT and semifluorinated compound) is approximately the same as the density of the aqueous phase. By adapting the density of the oil phase to the density of the aqueous phase coalescence phenomena of the droplets as well as sedimentation phenomena, which destabilize the emulsion, can be decreased.

Figure 1C shows the results of the density measurement of different mixtures of MCT and F6H8.

At about 38.5 wt.-% of F6H8 and about 61.5 wt.-% of MCT, (weight based on the total weight of the oil phase) the density of the oil mixture corresponds to the density of the aqueous phase.

### COMPARATIVE EXAMPLES 1-8, 9A and 10-12

Perfluoroctylbromide (PFOB) is a known contrast agent for the magnetic resonance spectroscopy. It has been found that PFOB cannot be dissolved in MCT. Therefore, emulsions have been prepared wherein the PFOB is stabilized by perfluorodecylbromide (PFDB) which can be dissolved in PFOB.

Table 3 and Table 4 shows the oil in water emulsion compositions of comparative examples 1-8, 9A and 10-12 and Table 6 shows the respective storage stability data at 20°C. The amounts referred to in Tables 2 and 4 are weight-% (wt.-%) based on the total weight of the emulsion.

**Table 3: Comparative Examples 1 to 4**

| Component | Comparative Example 1 | Comparative Example 2 | Comparative Example 3 | Comparative Example 4 |
|---|---|---|---|---|
| PFOB ¹⁾ | 20 | 20 | 20 | 20 |
| PFDB ²⁾ | 0.2 | 1 | 2 | 4 |
| Lipoid E 80 ³⁾ | 2 | 2 | 2 | 2 |
| Water | Ad 100 | Ad 100 | Ad 100 | Ad 100 |

| | | | | |
|---|---|---|---|---|
| ¹⁾ Perfluoroctylbromide ex ABCR GmbH & Co. KG, Germany ²⁾ Perfluordecylbromide ex ABCR GmbH & Co. KG, Germany ³⁾ Egg phospholipid ex Lipoid GmbH, Germany | | | | |

**Table 4: Comparative examples 5 to 8**

| Component | Comparative Example 5 | Comparative Example 6 | Comparative Example 7 | Comparative Example 8 |
|---|---|---|---|---|
| PFOB ¹⁾ | 20 | 20 | 20 | 20 |
| PFDB ²⁾ | 0.2 | 1 | 2 | 4 |
| Lipoid S PC-3 ³⁾ | 2 | 2 | 2 | 2 |
| Water | Ad 100 | Ad 100 | Ad 100 | Ad 100 |

| | | | | |
|---|---|---|---|---|
| ¹⁾ Perfluoroctylbromide ²⁾ Perfluordecylbromide ³⁾ Phospholipid ex Lipoid GmbH, Germany | | | | |

**Table 5: Comparative Examples 9A and 10 to 12**

| Component | Comparative Example 9A | Comparative Example 10 | Comparative Example 11 | Comparative Example 12 |
|---|---|---|---|---|
| PFOB ¹⁾ | 20 | 20 | 20 | 20 |
| PFDB ²⁾ | 0.2 | 1 | 2 | 4 |
| Lipoid S 75 ³⁾ | 2 | 2 | 2 | 2 |
| Water | Ad 100 | Ad 100 | Ad 100 | Ad 100 |

| | | | | |
|---|---|---|---|---|
| ¹⁾ Perfluoroctylbromide ex ABCR GmbH & Co. KG, Germany ²⁾ Perfluordecylbromide ex ABCR GmbH & Co. KG, Germany ³⁾ Soja phospholipid ex Lipoid GmbH, Germany | | | | |

**Table 6: Storage stability data of the emulsions according to Comparative examples 1 to 8, 9A and 10 to 12**

| Comparative Example | Storage time | ZAverage | P.I. | pH |
|---|---|---|---|---|
| | [days] | [nm] | | |
| 1 | 0 | 168,5 | 0,128 | 6,93 |
| | 7 | 314,3 | 0,197 | 5,59 |
| | 14 | 310,0 | 0,217 | 5,14 |
| | 28 | 316,5 | 0,242 | 4,52 |
| 2 | 0 | 119,7 | 0,187 | 6,75 |
| | 7 | 270,8 | 0,150 | 5,55 |
| | 14 | 278,4 | 0,221 | 5,35 |
| | 28 | 267,6 | 0,187 | 5,32 |
| 3 | 0 | 157,7 | 0,155 | 7,11 |
| | 7 | 249,0 | 0,156 | 5,85 |
| | 14 | 263,7 | 0,153 | 5,53 |
| | 28 | 178,4 | 0,061 | 5,74 |
| 4 | 0 | 157,5 | 0,193 | 6,99 |
| | 7 | 244,7 | 0,121 | 6,01 |
| | 14 | 267,5 | 0,120 | 5,71 |
| | 28 | 259,2 | 0,154 | 5,55 |
| 5 | 0 | 141,5 | 0,222 | 7,25 |
| | 7 | 318,8 | 0,160 | 6,47 |
| | 14 | 377,9 | 0,250 | 6,40 |
| | 28 | 485,8 | 0,358 | 6,04 |
| 6 | 0 | 130,1 | 0,282 | 7,27 |
| | 7 | 290,5 | 0,134 | 6,98 |
| | 14 | 444,3 | 0,224 | 6,62 |
| | 28 | 365,5 | 0,257 | 6,73 |
| 7 | 0 | 146,7 | 0,184 | 7,75 |
| | 7 | 269,0 | 0,171 | 6,96 |
| | 14 | 289,8 | 0,160 | 5,83 |
| | 28 | 315,4 | 0,156 | 6,56 |
| 8 | 0 | 141,9 | 0,217 | 7,58 |
| | 7 | 230,8 | 0,129 | 6,68 |
| | 14 | 253,0 | 0,117 | 6,69 |
| | 28 | 346,6 | 0,133 | 5,90 |
| 9A | 0 | 170,1 | 0,117 | 7,29 |
| | 7 | 272,8 | 0,114 | 6,38 |
| | 14 | 283,2 | 0,121 | 5,79 |
| | 28 | 249,6 | 0,071 | 5,23 |
| 10 | 0 | 152,4 | 0,137 | 7,18 |
| | 7 | 241,8 | 0,094 | 5,96 |
| | 14 | 233,5 | 0,100 | 5,59 |
| | 28 | 243,3 | 0,092 | 5,29 |
| 11 | 0 | 165,5 | 0,128 | 7,18 |
| | 7 | 217,5 | 0,115 | 6,04 |
| | 14 | 224,1 | 0,136 | 6,03 |
| | 28 | 224,8 | 0,096 | 5,66 |
| 12 | 0 | 144,9 | 0,135 | 7,24 |
| | 7 | 207,0 | 0,127 | 5,78 |
| | 14 | 212,4 | 0,092 | 5,97 |
| | 28 | 208,9 | 0,107 | 5,71 |

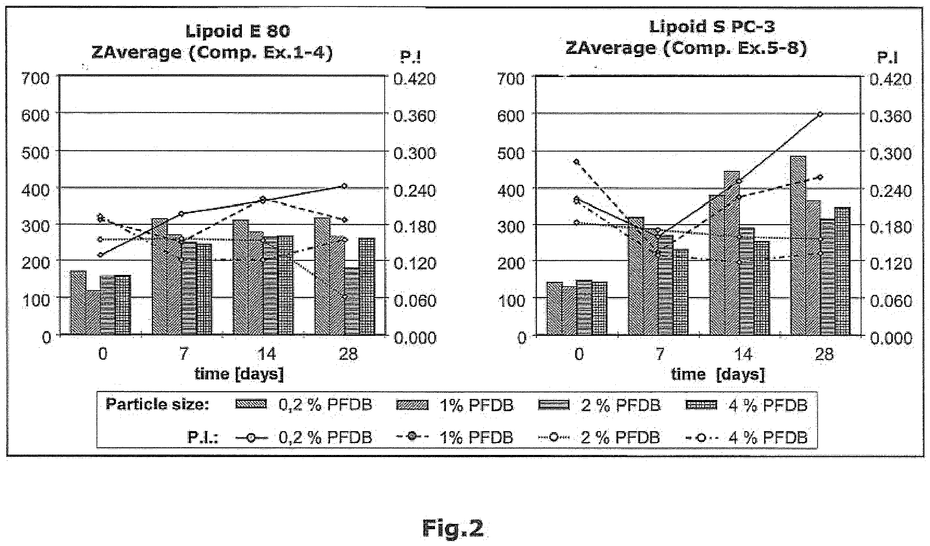

Figure 2 shows the data for the storage stability tests as reflected in Table 6. In the left part of Fig. 2 the data for the emulsions according to Comparative examples 1 to 4 and in the right part the the data for the emulsions according to Comparative examples 5 to 8 are depicted.

Figure 2A shows the data for the storage stability tests for Example 9A and 10 to 12.

As can be seen from Table 6 and Figure 2 as well as Fig. 2A the emulsions of comparative examples 1 to 8, 9A and 10 to 12 show a significant increase of the average particle size over a storage time of 28 days.

In contrast the emulsions according to examples 1 to 3 of the inventions are much more storage stable. During a storage time of 28 days only a slight increase in the average particle size of the emulsion could be observed (Fig. 1 and Table 2).

### Test with different emulsifier

It has been surprisingly found that the emulsions of the present invention can be further stabilized by the selection of the emulsifier.

The emulsifier Lipid S PC-3 is considered less suitable (Fig. 2) and has not been further tested for the emulsions of the invention.

Table 7 shows emulsions of the present invention according to examples 4 and 5.

**Table 7: Oil in water emulsion compositions according to examples 4 and 5**

| Component | Example 4 | Example 5 |
|---|---|---|
| F6H8 ¹⁾ | 8 | 8 |
| MCT²⁾ | 12 | 12 |
| Lipoid E 80 ³⁾ | 2 | ----- |
| Lipoid S 75 ⁴⁾ | ----- | 2 |
| Sodium oleate | 0.03 | 0.03 |
| alpha-tocopherol | 0.02 | 0.02 |
| Water | Ad 100 | Ad 100 |

| | | |
|---|---|---|
| ¹⁾ Perfluorhexyloctane ex Novaliq, Germany ²⁾ Mid chain triglycerides (50 - 80 wt.-% C₈-fatty acid; 20 - 50 wt.-% C₁₀-fatty acid) ³⁾ Egg Phospholipid ex Lipoid GmbH, Germany ⁴⁾ Phospholipid ex Lipoid GmbH, Germany (soya phospholipid with 14 to 25 wt.-% glycolipid) | | |

The emulsions according to example 4 and example 5 are further analysed whether sterilization conditions effect the stability of the emulsions. The emulsions have been sterilized in a rotating autoclave and are compared with not sterilized emulsions (sterilization condition : heating at 121°C for 15 min at 2 bar).

Table 8 shows the stability data of the emulsions according to example 4 and 5 without sterilization and after sterilization.

**Table 8: Storage stability data for examples 4 and 5 of the invention under sterilized and not sterilized conditions**

| Example | Sterilized/ Not sterilized | Storage time | ZAverage | P.I. | pH |
|---|---|---|---|---|---|
| | | [days] | [nm] | | |
| 4 | Not sterilized | 0 | 156,5 | 0,082 | 8,13 |
| | | 7 | 170,5 | 0,108 | 5,84 |
| | | 14 | 176,6 | 0,086 | 5,68 |
| | | 28 | 194,9 | 0,043 | 5,00 |
| | sterilized | 0 | 156,5 | 0,082 | 8,13 |
| | | 7 | 263,2 | 0,028 | 7,15 |
| | | 14 | 264,9 | 0,072 | 7,12 |
| | | 28 | 260,8 | 0,040 | 7,07 |
| 5 | Not sterilized | 0 | 170,6 | 0,102 | 8,00 |
| | | 7 | 176,9 | 0,080 | 6,40 |
| | | 14 | 177,7 | 0,075 | 5,12 |
| | | 28 | 167,0 | 0,067 | 4,07 |
| | sterilized | 0 | 170,6 | 0,102 | 8,00 |
| | | 7 | 177,0 | 0,059 | 8,09 |
| | | 14 | 178,9 | 0,095 | 8,00 |
| | | 28 | 175,4 | 0,086 | 7,93 |

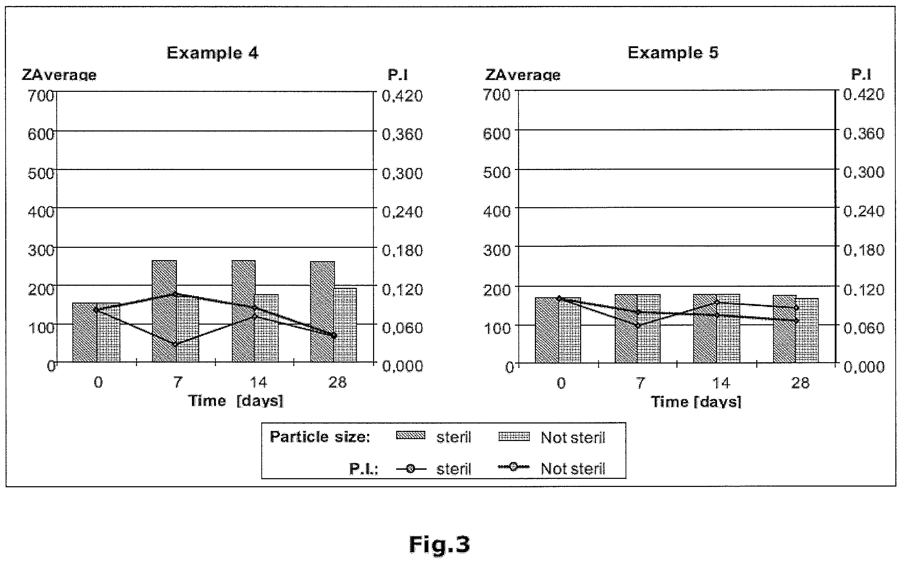

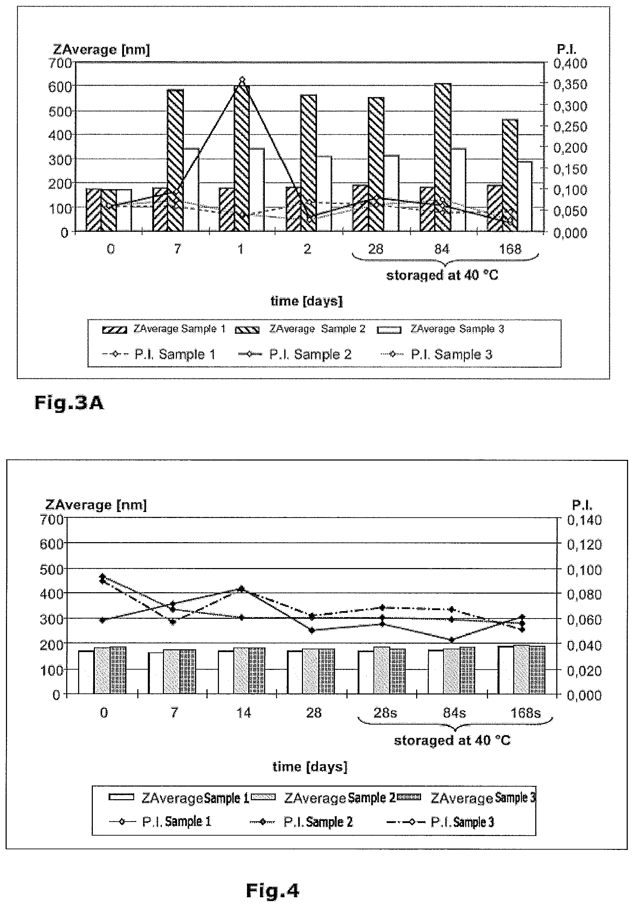

Figure 3 shows the data for the storage stability tests as reflected in Table 8. In the left part of Fig. 3 the data for the emulsions according to example 4 and in the right part the data for the emulsions according to example 5 are depicted.

Figure 3 and Table 8 show that the emulsion according to example 5 is more stable than the emulsion of example 4. The emulsion according to example 5 is also more stable after the emulsion has been sterilized in a rotating autoclave.

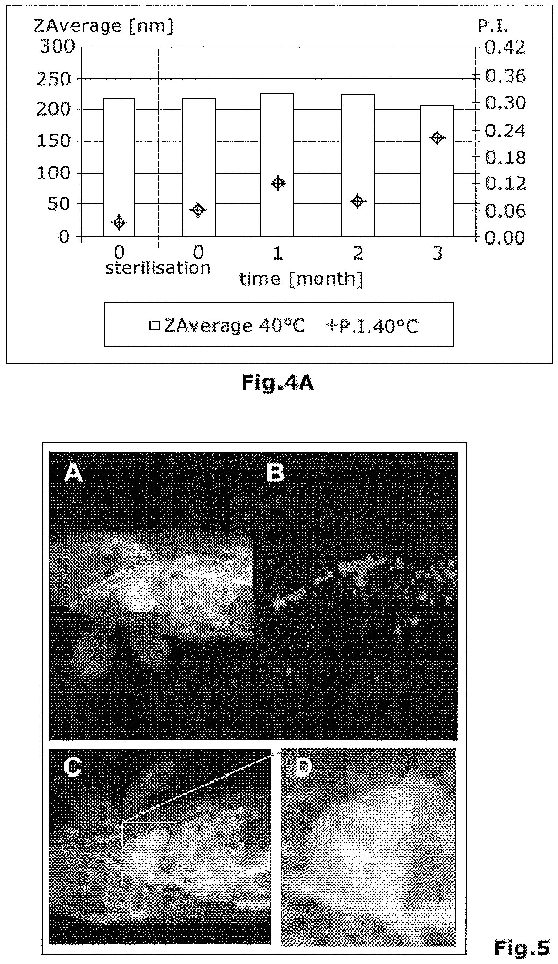

Further the emulsions of example 4 and 5 have been stored for 168 days at 40 °C. It has surprisingly found that the emulsion of example 5 even at a storage temperature of 40 °C did not significantly change the physical stability (Fig. 4).

Fig. 4 shows the test results of 3 samples of the emulsion according to example 5.

Fig. 3A shows the test results of 3 samples of the emulsion according to example 4.

### Example 6 of the invention:

**Table 9: Emulsion composition according to Example 6**

| Component | Example 6 |
|---|---|
| F6H8 | 8 |
| MCT | 12 |
| Lipoid S 75 | 2 |
| alpha-tocopherol | 0.02 |
| Glycerol | 2.5 |
| Sodium oleate | 0.03 |
| NaOH | Ad pH 8.2 |
| Water | Ad 100 |

The emulsion according to example 6 has been prepared by preparing a homogenous mixture of F6H8, MCT and alpha-tocoperhol at 50 °C. Separately, a mixture of the emulsifier Lipoid S 75 and the co-emulsifier sodium oleate together with glycerol and water are mixed in a rotor/stator mixing device (Ultra turrax) at 50 °C at 15000 rpm for 20 minutes. Subsequently, the mixture comprising MCT, F6H8 and alpha-tocopherol is given to the aqueous mixture and homogenized at 50 °C with an Ultra turrax (type T 25 ex IKA) at 20000 rpm for 20 minutes. The pre-emulsion obtained is subsequently further homogenized in a high pressure homogenizer (APV type 1000/2000) [five cycles at 500 bar and subsequently at 60 bar].

The emulsion obtained is subsequently sterilized at 121 °C for 15 minutes in an autoclave.

It has surprisingly found that the emulsion of example 6 even at a storage temperature of 40 °C did not significantly change the physical stability even after three months (Fig. 4A).

Figure 4A shows the test results of the emulsion according to example 6.

The emulsion composition of example 6 has been used for in vivo MRI measurements.

In order to check whether the F6H8/MCT emulsions can be visualized in vivo by means of MRI and detected in the target tissue, first pharmacokinetic examinations were performed.

Figure 5 shows Blood pool effects in an exemplary manner.

Figure 5 shows emulsion 6 according of the invention in the heart as the target tissue in Blood pool. Exemplary 3D reconstructions of the anatomic T2 TSE and the colored 3D ¹⁹F sequences have been reconstructed commonly in one data set from a rat as a total data set (A) and with the liver removed by computing (B). In the row below, the complete 3D data set (C) with the heart as an enlarged detail (D) is shown.

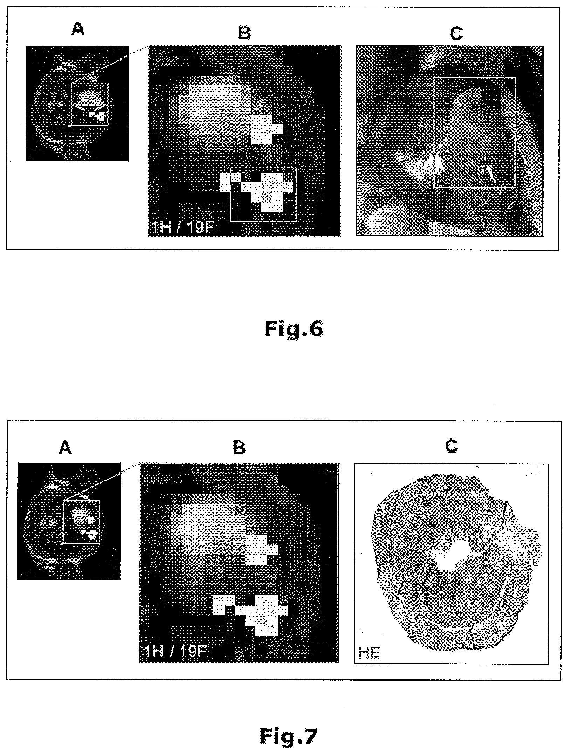

Thus, it can be shown that the emulsions according to the invention can be visualized in vivo in the myocardium. Further, the representation of an infarction in MRI was examined. The results are shown in the following Figure 6.

Figure 6 shows: (A) Rat thorax in transversal section in the myocardial area in vivo in a T2-weighted MRI image (1.5 Tesla, Philips), which is superimposed with the colored 19F image (1H/19F), with the same geometry 24 h after the application of the emulsion in accordance with Example 6. (B) The myocardium as an enlarged detail of the 1H/19F image, which shows the enrichment of the fluorine-containing contrast agent on the level of the myocardial infarction, and the site of adhesion between the pericardium and the ribs caused by the surgery. (C) In situ recording of the rat heart, which shows a strongly pronounced inflammation of the pericardium (green outline) beside the infarcted area.

Thus, it could be shown that the contrast agent becomes enriched in the infarcted area. Further, the MRI images were compared with the histological sections, which is shown in Figure 7.

Figure 7: (A) Rat thorax in transversal section in the myocardial area in vivo in a T2-weighted MRI image (1.5 Tesla, Philips), which is superimposed with the colored 19F image (1H/19F), with the same geometry 24 h after the application of the emulsion in accordance with Example 6. (B) The myocardium as an enlarged detail of the 1H/19F image, which shows the enrichment of the fluorine-containing contrast agent on the level of the myocardial infarction, and the site of adhesion between the pericardium and the ribs caused by the surgery. (C) HE staining of an exemplary histological short-axis section [7 µm] of the infarcted rat myocardium shown under A. The infarcted myocardial area is shown in blue in HE staining.

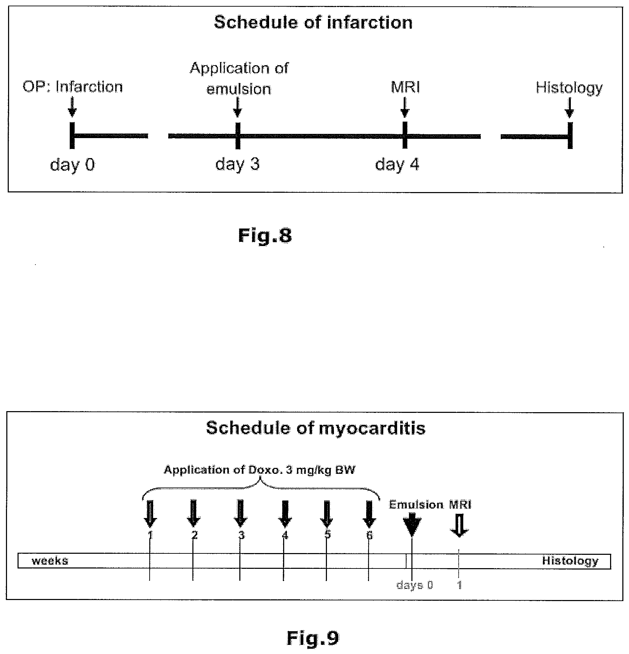

The emulsions have been tested on two models:
the myocard infarct model the time line of which is reflected in Fig. 8; and
the myocarditis model the time of which is reflected in Fig. 9.

### Induction of infarction

As animals SD-rats having a weight of 200 to 350 g have been used.
Anesthesia: These animals are sedated in an anesthesia box by isoflurane inhalation, and then anesthetized by an intramuscular administration. Anesthesia that ensures freedom from pain even during the anesthesia is to be guaranteed. Therefore, the animals obtain a mixture of anesthesia and analgesia with: 0.15 mg/kg medetomidine + 0.05 mg/kg fentanyl + 2.0 mg/kg midazolam i.m.; the antagonization is effected with 0.75 mg/kg atipamezole + 0.12 mg/kg naloxone + 0.2 mg/kg s.c. Because of our previous experiences with flunixine (2.5 mg/kg s.c.), we shall employ it as a preparation for postoperative pain therapy. If the therapy fails, we shall consider a modification of the analgesic therapy with buprenorphine (0.05 mg/kg s.c.).

Subsequently, the animal obtains an i.m. atropine administration during the subsequent intubation for supporting the circulation and for protection against vagal irritations.

Fluorine-based contrast agents in cardiovascular diseases:
Even if the use of medetomidine is recommendable basically only for animals with a healthy cardiovascular system, it must be expected in healthy young animals (the planned cardiothoracic measures are performed with animals having a body weight of about 200 g) that the Frank Starling mechanism is not yet fully ripened. Therefore, the bradycardia mediated by the central-antisympathicotonic activity of medetomidine may not be sufficiently compensated by an increased stroke volume. The administration of atropine causes an increase of cardiac work. However, the vasoconstriction triggered by peripheral α2 receptor stimulation does not change the coronary (as well as cerebral and renal) blood flow substantially, so that a specific cardiac load is not to be assumed here, and the chronotropic effect of atropine for the overall situation of the circulation should be predominant. It is therefore considered that a transient hypertensive circulatory condition should be well tolerated, which is in accordance with clinical observations in the postoperative phase. Additionally always take coronary ligation and its possible (acute) effect on the pumping performance of the heart is taken into account. However, because of the complexity of the manual operation and the variance of coronary anatomy of the test animals, a universal law cannot be formulated.

The hypertensive effects are subsiding immediately after the postoperative antagonization of medetomidine, so that the duration of the hypertension remains within reasonable limits and, above all, controllable.

Since the animals are intubated in the context of the operative measure, there are risks of vagal irritations (with consecutive bradycardia) on the one hand, and of complicated intubation in the case of hypersalivation on the other; the latter is also consistent with our own experiences. These risks can be reduced by a preoperative administration of atropine.

All in all, an indication for preoperative atropine administration was found.

After the tolerance stage has begun, the rat is shaved and disinfected on the left side.

The rat is subsequently connected to the respiration pump, which is set to a frequency of 75-80 strokes per minute and a tidal volume of 2.0 ml (for rats weighing 200-250 g). It is advisable to connect the animal to the respiration pump as late as possible before the thoracotomy is started, because artificial respiration always means another intervention in the organism.

After the respiration pump is connected, the thorax can be opened with a curved forceps on the level of the 5th and 6th ribs. This should be done with utmost caution, because the lung can be injured very easily thereby. The thoracic cavity must be opened over a length of about 1.0 cm to 1.5 cm for the wound retractor to fit in. As soon as a sufficiently large operating area has been created, optionally by a corresponding soft-tissue dissection, the course of the arteria coronaria sinistra must be detected. It originates from the aorta between the pulmonary trunk and the left atrial appendage, easily recognized by an epicardial fat line. However, the arteria coronaria sinistra often cannot be seen, or only partly so, because the coronary vessels of rats do not run superficially, but within the myocardium. The main branch of the arteria coronaria sinistra, the LAD (left anterior descending), runs substantially straight from the aortic root to the apex of the heart. One of the proximal side branches (D1 or D2) of the LAD is shut off by a coronary ligature. The suture-ligature of the D1 or D2 side branch is effected by a single-knot suture. An occlusion of the (proximal) LAD itself would cause too extended an infarction scenario, which is only rarely survived by the animals. Subsequently, the thoracic cage is closed in two layers (rib and skin sutures). The ribs are closed by two or three single-knot sutures. The chest muscles are merely laid on top of each other. The skin seam is closed by a continuous glover's suture.

3 days after the ligature the fluorocarbon containing emulsions are administered to the aminals. After further 24 hours MRI analysis is performed (see Fig. 8)
All animals are given analgesia every 12 hours over the entire test duration of 6 days: flunixine (Finadyne) 2.5 mg/kg of body mass s.c.

General data of development, such as weight and behavior, are acquired from all animals twice a week.

### Infarction surgery:

These animals are sedated in an anesthesia box by isoflurane inhalation, and then anesthetized by an intramuscular administration of a mixed Domitor^{®}/Dormicum^{®} solution (0.3 mg of medetomidine and 4 mg of midazolam per kg of body weight). Subsequently, the animals obtain an atropine administration (0.01 mg atropine per kg of body mass, s.c.) during the subsequent intubation for supporting the circulation and for protection against vagal irritations.

### MRI examinations:

These animals exclusively obtain an inhalation anesthesia (oxygen + isoflurane 2.0%). The latter is well tolerable, and the animals have no long waking phase afterwards.

While under anesthesia, the animals have been administered the fluorine-based contrast agents through the tail vein.

### Inflammation model of the myocardium:

All animals obtain an i.v. administration of doxorubicin or isotonic saline solution once a week over a period of 6 consecutive weeks. Each administration is effected under general anesthesia. One week after the last administration the animals are administered with the emulsion of the invention. 24 hours later the MRI analysis is performed.

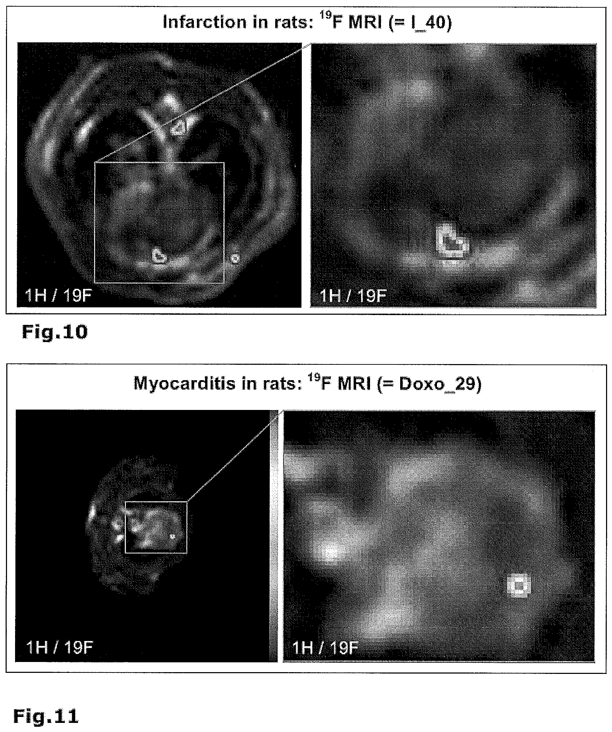

The emulsion according to Example 6 has been modified in that the emulsifier has been replaced by an pegylated phospholipid. It has been found that the opsonation of the emulsion droplets are significantly reduced. Due to this effect it was expected that the amount of fluorocarbon compound (F6H8) in the area of inflammation is below the detection limit. However, it has surprisingly found that the myocarditis could even be imaged which is shown in Fig. 11.

On the days of examination in the MRI, the animals obtain an inhalation anesthesia (isoflurane and oxygen mixture 1.5%-2.5%). Further, the animals are administered a dose of the fluorine-based contrast agent compounds through the tail vein during the examination depending on the group to which they belong. After the last MRI examination, the respective animal is sacrificed by providing an overdose of isoflurane, and the organs are subsequently removed.

All animals additionally obtain an analgesia. For this purpose, paracetamol AL is employed, because it has only a little anti-inflammatory effect. The animals obtain the paracetamol through the drinking water at a dosage of 150 mg/kg of body weight over the entire experimental period.

General data of development, such as weight and behavior, are acquired *daily* from all animals.

### Induction of myocarditis:

The animals exclusively obtain an inhalation anesthesia (oxygen + isoflurane 2.0% + laughing gas). The latter is well tolerable and does not lead to a prolonged waking phase. With the initiation of the anesthesia, the animals obtain a corresponding analgesia.

### MRI examinations:

The animals exclusively obtain an inhalation anesthesia (oxygen + isoflurane 2.0%). The latter is well tolerable and does not lead to a prolonged waking phase. While under anesthesia, the animals are administered a fluorine-based contrast agent through the tail vein.

The MR results of an infarct of an animal treated with the composition of Example 6 is reflected in Fig. 10 and of a myocarditis wherein the animal is treated with an emulsion as described in Example 6 with the exception that the emulsifier is replaced by an pegylated phospholipid is reflected in Fig. 11.

## Claims

1. Aqueous emulsion comprising
a) a semifluorinated compound of formula I:
CF₃-(CF₂)ₓ-(CH₂)_{y}-CH₃ (I)
wherein x is an integer ranging from 1 to 8 and y is an integer ranging from 2 to 10,
b) a triglyceride which is miscible with the semifluorinated compound at 20 °C; and
c) an emulsifier.

2. Emulsion according to claim 1 wherein the semifluorinated compound is perfluorohexyloctane (F6H8) and/or perfluorbutylpentane (F4H5).

3. Emulsion according to one or more of the preceding claims wherein the amount of the semifluorinated carbon compound a) and the triglyceride b), which is preferable a mid-chain triglyceride, ranges from 5 to 40 wt.-%, preferably 10 to 30 wt.-%, especially 15 to 25 wt.-%, based on the total weight of the emulsion.

4. Emulsion according to one or more of the preceding claims wherein the weight ratio of the semifluorinated carbon compound a) to the triglyceride b), which is preferably a mid-chain triglyceride (MCT), ranges from 1:20 to 1:0.20, preferably 1:15 to 1:0.25, more preferably 1:10 to 1:0.30, further preferably 1:4 to 1:0.35, especially 1:2 to 1:0.4.

5. Emulsion according to one or more of the preceding claims for use as a contrast agent or contrast enhancing agent.

6. Emulsion according to one or more of the preceding claims for use as a contrast enhancement agent or contrast agent for the diagnostic detection, by means of an imaging procedure utilizing MR-techniques.

7. Emulsion according to one or more of claims 1 to 5 for use in the diagnostic detection, by means of an imaging procedure, of inflammatory processes selected from the group consisting of inflammatory reaction peripheral to infarctions such as myocardial infarction, stroke; inflammation of organs, such as myocarditis, encephalitis, meningitis; multiple sclerosis; inflammation of the gastrointestinal tract, such as Crohn's disease; inflammation of the vessels, such as arteriosclerosis, in particular vulnerable plaques; detection of abscesses and also arthritis, wherein the imaging process is based on measuring the nuclear magnetic resonance of the ¹⁹F isotope.

8. Emulsion according to one or more of claims 1 to 5 for use in the diagnostic detection, based on non-invasive imaging procedures of the cardio-vascular system, including the myocardium, arteries and veins; inflammatory reactions occurring in disease processes like myocardial infarction, myocarditis, atherosclerosis and thrombosis leading to inflammatory and degenerative processes of the vasculature found in neurology such as stroke or tumor; pulmology such as thrombosis, inflammation, sarcoidosis; gastroenterology such as tumour, inflammatory bowl diseases such as Crohn's disease; and rheumatology such as autoimmune diseases of the vessels such as Takayasu arteritis.

9. Use of the emulsion according to claims 1 to 5 in a magnetic resonance imaging procedure.

10. Method for the manufacturing of the emulsion according to one or more of claims 1 to 8 comprising the steps:
a) preparing a mixture by mixing
- a semifluorinated compound of formula I:
CF₃-(CF₂)ₓ-(CH₂)_{y}-CH₃ (I)
wherein x is an integer ranging from 1 to 8 and y is an integer ranging from 2 to 10,
- a triglyceride which is miscible with the semifluorinated compound at 20 °C,
- an emulsifier; and
- water
b) homogenizing the mixture obtained in step a); and
c) optionally sterilizing the emulsion.

11. Method according to claim 10 comprising the following steps:
a) preparing a mixture by
a-1) preparing a mixture comprising water and an emulsifier,
a-2) preparing a homogenous mixture comprising the semifluorinated compound and the triglyceride,
a-3) combining the mixture obtained in step a-1) with the mixture obtained in step a-2); and
b) homogenizing the mixture obtained in step a), preferably homogenizing the mixture obtained in step a) with a high pressure homogenizer.

12. Method for operating a magnetic resonance tomograph for the detection of a semifluorinated compound of formula I:
CF₃-(CF₂)ₓ-(CH₂)_{y}-CH₃ (I)
wherein x is an integer ranging from 1 to 8 and y is an integer ranging from 2 to 10,
in an emulsion as defined in one or more of claims 1 to 8, the method comprising the method steps:
- emitting an alternating magnetic field,
- detecting an absorption of the emitted magnetic field by a sample to be examined,
**characterized in that**
the frequency of the alternating magnetic field is between H ▪ 40.05650 MHz/T and H ▪ 40.06150 MHz/T, H being the magnetic field strength in Tesla.

13. Method for detecting in a sample to be examined a semifluorinated compound of formula I:
CF₃-(CF₂)ₓ-(CH₂)_{y}-CH₃ (I)
wherein x is an integer ranging from 1 to 8 and y is an integer ranging from 2 to 10 in an emulsion according to one or more of claims 1 to 8,
the method comprising the following method steps:
- emitting an alternating magnetic field,
- detecting an absorption of the emitted magnetic field by a sample to be examined,
**characterized by** the following method steps:
- superimposing at least one ¹⁹F-fluorine proton image over a ¹H proton
image,
whereby the protons of ¹H
image is displayed as a grey scale image and the protons of ¹⁹F fluorine image is displayed by using a color gradient, one end of this color gradient indicating a low concentration of ¹⁹F and the other end of this color gradient indicating a high concentration of ¹⁹F.

14. Method according to claim 13 **characterized by** the following method steps:
- eliminating disturbing signals generated by ¹⁹F fluorine protons in a second sample not to be examined by the following method steps:
- comparing the value for the fluorine concentration in a first pixel with values of all pixels in the three-dimensional space around this first pixel,
whereby, if the difference between the values for the fluorine concentration of two adjacent pixels lies below a threshold these two pixels are considered to belong to a cluster of pixels,
- removing this disturbing cluster of pixels from the image obtained, if the cluster does not represent the sample to be examined.

15. Method comprising acquiring non-invasive imaging procedures of a patient to whom the emulsion according to one or more of claims 1 to 8 has previously administered so as to dissolve it in the patient's bloodstream.

## Patentansprüche

1. Wässrige Emulsion, umfassend
a) eine semifluorierte Verbindung der Formel I:
CF₃-(CF₂)ₓ-(CH₂)_{y}-CH₃ (I),
wobei x eine ganze Zahl im Bereich von 1 bis 8 ist und y eine ganze Zahl im Bereich von 2 bis 10 ist;
b) ein Triglycerid, das bei 20 °C mit der semifluorierten Verbindung mischbar ist; und
c) einen Emulgator.

2. Emulsion gemäß Anspruch 1, wobei es sich bei der semifluorierten Verbindung um Perfluorhexyloctan (F6H8) und/oder Perfluorbutylpentan (F4H5) handelt.

3. Emulsion gemäß einem oder mehreren der vorstehenden Ansprüche, wobei die Menge der semifluorierten Kohlenstoffverbindung a) und des Triglycerids b), das vorzugsweise ein mittelkettiges Triglycerid ist, im Bereich von 5 bis 40 Gew.-%, vorzugsweise 10 bis 30 Gew.-%, insbesondere 15 bis 25 Gew.-%, liegt, bezogen auf das Gesamtgewicht der Emulsion.

4. Emulsion gemäß einem oder mehreren der vorstehenden Ansprüche, wobei das Gewichtsverhältnis der semifluorierten Kohlenstoffverbindung a) zu dem Triglycerid b), das vorzugsweise ein mittelkettiges Triglycerid (MCT) ist, im Bereich von 1:20 bis 1:0,20, vorzugsweise 1:15 bis 1:0,25, besonders bevorzugt 1:10 bis 1:0,30, weiter bevorzugt 1:4 bis 1:0,35, insbesondere 1:2 bis 1:0,4, liegt.

5. Emulsion gemäß einem oder mehreren der vorstehenden Ansprüche zur Verwendung als Kontrastmittel oder kontrastverstärkendes Mittel.

6. Emulsion gemäß einem oder mehreren der vorstehenden Ansprüche zur Verwendung als kontrastverstärkendes Mittel oder Kontrastmittel für den diagnostischen Nachweis mittels eines Bildgebungsverfahrens unter Verwendung von MR-Techniken.

7. Emulsion gemäß einem oder mehreren der Ansprüche 1 bis 5 zur Verwendung beim mittels eines Bildgebungsverfahrens erfolgenden diagnostischen Nachweis von Entzündungsvorgängen, die aus der Gruppe ausgewählt sind, die aus einer entzündlichen Reaktion im Peripheriebereich von Infarkten, wie Myokardinfarkt oder Schlaganfall, einer Entzündung von Organen, wie Myokarditis, Enzephalitis oder Meningitis, multipler Sklerose, einer Entzündung des Magen-Darm-Trakts, wie Morbus Crohn, einer Entzündung der Blutgefäße, wie Arteriosklerose, insbesondere vulnerable Plaques, dem Nachweis von Abszessen und auch Arthritis besteht, wobei das Bildgebungsverfahren auf der Messung der kernmagnetischen Resonanz des ¹⁹F-Isotops beruht.

8. Emulsion gemäß einem oder mehreren der Ansprüche 1 bis 5 zur Verwendung beim diagnostischen Nachweis, der auf nichtinvasiven Bildgebungsverfahren des kardiovaskulären Systems einschließlich des Myokards, der Arterien und Venen beruht, von Entzündungsreaktionen, die bei Krankheitsvorgängen wie Myokardinfarkt, Myokarditis, Atherosklerose und Thrombose auftreten und zu entzündlichen und degenerativen Vorgängen des Gefäßsystems führen, die man in der Neurologie, wie Schlaganfall oder Tumor, Pulmologie, wie Thrombose, Entzündung, Sarkoidose, Gastroenterologie, wie Tumor, entzündlichen Darmerkrankungen, wie Morbus Crohn, und Rheumatologie, wie Autoimmunerkrankungen der Blutgefäße, wie Takayasu-Arteriitis, findet.

9. Verwendung der Emulsion gemäß den Ansprüchen 1 bis 5 in einem Magnetresonanz-Bildgebungsverfahren.

10. Verfahren zur Herstellung der Emulsion gemäß einem oder mehreren der Ansprüche 1 bis 8, umfassend die Schritte:
a) Herstellen eines Gemischs durch Mischen von
- einer semifluorierten Verbindung der Formel I:
CF₃-(CF₂)ₓ-(CH₂)_{y}-CH₃ (I),
wobei x eine ganze Zahl im Bereich von 1 bis 8 ist und y eine ganze Zahl im Bereich von 2 bis 10 ist;
- einem Triglycerid, das bei 20 °C mit der semifluorierten Verbindung mischbar ist;
- einem Emulgator; und
- Wasser;
b) Homogenisieren des in Schritt a) erhaltenen Gemischs; und
c) gegebenenfalls Sterilisieren der Emulsion.

11. Verfahren gemäß Anspruch 10, umfassend die folgenden Schritte:
a) Herstellen eines Gemischs durch
a-1) Herstellen eines Gemischs, das Wasser und einen Emulgator umfasst;
a-2) Herstellen eines homogenen Gemischs, das die semifluorierte Verbindung und das Triglycerid umfasst;
a-3) Kombinieren des in Schritt a-1) erhaltenen Gemischs mit dem in Schritt a-2) erhaltenen Gemisch; und
b) Homogenisieren des in Schritt a) erhaltenen Gemischs, vorzugsweise Homogenisieren des in Schritt a) erhaltenen Gemischs mit einem Hochdruckhomogenisator.

12. Verfahren zum Betreiben eines Magnetresonanz-Tomographen zum Nachweis einer semifluorierten Verbindung der Formel I:
CF₃-(CF₂)ₓ-(CH₂)_{y}-CH₃ (I),
wobei x eine ganze Zahl im Bereich von 1 bis 8 ist und y eine ganze Zahl im Bereich von 2 bis 10 ist;
in einer Emulsion gemäß einem oder mehreren der Ansprüche 1 bis 8, wobei das Verfahren die folgenden Verfahrensschritte umfasst:
- Emittieren eines magnetischen Wechselfelds;
- Nachweisen einer Absorption des emittierten Magnetfelds durch eine zu untersuchende Probe;
**dadurch gekennzeichnet, dass**
die Frequenz des magnetischen Wechselfelds zwischen H • 40,05650 MHz/T und H • 40,06150 MHz/T liegt, wobei H die magnetische Feldstärke in Tesla ist.

13. Verfahren zum Nachweis einer semifluorierten Verbindung der Formel I in einer zu untersuchenden Probe:
CF₃-(CF₂)ₓ-(CH₂)_{y}-CH₃ (I),
wobei x eine ganze Zahl im Bereich von 1 bis 8 ist und y eine ganze Zahl im Bereich von 2 bis 10 ist, in einer Emulsion gemäß einem oder mehreren der Ansprüche 1 bis 8, wobei das Verfahren die folgenden Verfahrensschritte umfasst:
- Emittieren eines magnetischen Wechselfelds;
- Nachweisen einer Absorption des emittierten Magnetfelds durch eine zu untersuchende Probe;
**gekennzeichnet durch** die folgenden Verfahrensschritte:
- Überlagern wenigstens eines ¹⁹F-Fluor-Protonenbilds über ein ¹H-Protonenbild;
wobei die Protonen des ¹H-Bilds als Graustufenbild angezeigt werden und die Protonen des ¹⁹F-Fluor-Bilds unter Verwendung eines Farbgradienten angezeigt werden, wobei ein Ende dieses Farbgradienten eine geringe Konzentration an ¹⁹F anzeigt und das andere Ende dieses Farbgradienten eine hohe Konzentration an ¹⁹F anzeigt.

14. Verfahren gemäß Anspruch 13, **gekennzeichnet durch** die folgenden Verfahrensschritte:
- Eliminieren störender Signale, die **durch** ¹⁹F-Fluorprotonen in einer zweiten, nicht zu untersuchenden Probe erzeugt werden, **durch** die folgenden Verfahrensschritte:
- Vergleichen des Werts für die Fluorkonzentration in einem ersten Pixel mit Werten aller Pixel im dreidimensionalen Raum um diesen ersten Pixel herum;
wobei, wenn die Differenz zwischen den Werten für die Fluorkonzentration von zwei benachbarten Pixeln unterhalb einer Schwelle liegt, davon ausgegangen wird, dass diese beiden Pixel zu einem Haufen von Pixeln gehören;
- Entfernen dieses störenden Haufens von Pixeln aus dem erhaltenen Bild, wenn der Haufen nicht die zu untersuchende Probe repräsentiert.

15. Verfahren, umfassend das Erfassen von nichtinvasiven Bildgebungsverfahren bei einem Patienten, dem zuvor die Emulsion gemäß einem oder mehreren der Ansprüche 1 bis 8 verabreicht wurde, um sie im Blutstrom des Patienten aufzulösen.

## Revendications

1. Emulsion aqueuse, comprenant
a) un composé semifluoriné de la formule I :
CF₃-(CF₂)ₓ-(CH₂)_{y}-CH₃ (I),
où x est un nombre entier allant de 1 à 8, et y est un nombre entier allant de 2 à 10,
b) un triglycéride qui est miscible avec le composé semifluoriné à 20 °C, et
c) un émulsifiant.

2. Emulsion selon la revendication 1, dans laquelle le composé semifluoriné est le perfluorohexyloctane (F6H8) et/ou le perfluorobutylpentane (F4H5).

3. Emulsion selon une ou plusieurs des revendications précédentes, dans laquelle la quantité du composé de carbone semifluoriné a) et du triglycéride b), qui est de préférence un triglycéride à chaîne moyenne, est comprise entre 5 et 40 % en poids, de préférence entre 10 et 30 % en poids, notamment entre 15 et 25 % en poids, par rapport au poids total de l'émulsion.

4. Emulsion selon une ou plusieurs des revendications précédentes, dans laquelle le rapport pondéral du composé de carbone semifluoriné a) au triglycéride b), qui est de préférence un triglycéride à chaîne moyenne (MCT), est comprise entre 1:20 et 1:0,20, de préférence entre 1:15 et 1:0,25, de préférence encore entre 1:10 et 1:0,30, de préférence encore entre 1:4 et 1:0,35, notamment entre 1:2 et 1:0,4.

5. Emulsion selon une ou plusieurs des revendications précédentes, destinée à être utilisée comme agent de contraste ou agent de renforcement de contraste.

6. Emulsion selon une ou plusieurs des revendications précédentes, destinée à être utilisée comme agent de renforcement de contraste ou agent de contraste pour la détection diagnostique au moyen d'un procédé d'imagerie en utilisant des techniques de résonance magnétique (RM).

7. Emulsion selon une ou plusieurs des revendications 1 à 5, destinée à être utilisée dans la détection diagnostique, au moyen d'un procédé d'imagerie, de processus inflammatoires choisis dans le groupe consistant en une réaction inflammatoire à la périphérie d'infarctus, telle qu'un infarctus du myocarde, un accident vasculaire cérébral ; une inflammation d'organes, telle que la myocardite, l'encéphalite, la méningite ; la sclérose en plaques ; une inflammation du tractus gastro-intestinal, telle que la maladie de Crohn ; une inflammation des vaisseaux sanguins, telle que l'artériosclérose, notamment des plaques vulnérables ; la détection d'abcès et encore l'arthrite, dans laquelle ledit procédé d'imagerie est basé sur la mesure de la résonance magnétique nucléaire de l'isotope ¹⁹F.

8. Emulsion selon une ou plusieurs des revendications 1 à 5, destinée à être utilisée dans la détection diagnostique, basée sur des procédés d'imagerie non invasives du système cardiovasculaire, incluant le myocarde, des artères et des veines ; des réactions inflammatoires survenues dans des processus pathologiques, comme l'infarctus du myocarde, la myocardite, l'athérosclérose et la thrombose entraînant des processus inflammatoires et dégénératifs du système vasculaire trouvés dans la neurologie, comme un accident vasculaire cérébral ou une tumeur ; dans la pulmologie, comme la thrombose, l'inflammation, la sarcoïdose ; dans la gastroentérologie, comme une tumeur, des maladies inflammatoires de l'intestin, comme la maladie de Crohn ; et dans la rhumatologie, comme des maladies auto-immunes des vaisseaux, comme l'artérite de Takayasu.

9. Utilisation de l'émulsion selon les revendications 1 à 5 dans un procédé d'imagerie par résonance magnétique.

10. Procédé pour produire l'émulsion selon une ou plusieurs des revendications 1 à 8, comprenant les étapes consistant à :
a) préparer un mélange en mélangeant
- un composé semifluoriné de la formule I :
CF₃-(CF₂)ₓ-(CH₂)_{y}-CH₃ (I),
où x est un nombre entier allant de 1 à 8, et y est un nombre entier allant de 2 à 10,
- un triglycéride qui est miscible avec le composé semifluoriné à 20 °C,
- un émulsifiant, et
- de l'eau,
b) homogénéiser le mélange obtenu dans l'étape a), et
c) éventuellement stériliser l'émulsion.

11. Procédé selon la revendication 10, comprenant les étapes suivantes consistant à :
a) préparer un mélange en
a-1) préparant un mélange comprenant de l'eau et un émulsifiant,
a-2) préparant un mélange homogène comprenant le composé semifluoriné et le triglycéride,
a-3) combiner le mélange obtenu dans l'étape a-1) avec le mélange obtenu dans l'étape a-2), et
b) homogénéiser le mélange obtenu dans l'étape a), de préférence homogénéiser le mélange obtenu dans l'étape a) avec un homogénéisateur à haute pression.

12. Procédé pour gérer un tomographe à résonance magnétique pour la détection d'un composé semifluoriné de la formule I :
CF₃-(CF₂)ₓ-(CH₂)_{y}-CH₃ (I),
où x est un nombre entier allant de 1 à 8, et y est un nombre entier allant de 2 à 10,
dans une émulsion telle que définie dans une ou plusieurs des revendications 1 à 8, le procédé comprenant les étapes du processus suivantes consistant à :
- émettre un champ magnétique alternatif,
- détecter une absorption du champ magnétique émis, par un échantillon à examiner,
**caractérisé en ce que** la fréquence du champ magnétique alternatif est comprise entre H • 40,05650 MHz/T et H • 40,06150 MHz/T, où H est l'intensité de champ magnétique en Tesla.

13. Procédé pour détecter dans un échantillon à examiner un composé semifluoriné de la formule I :
CF₃-(CF₂)ₓ-(CH₂)_{y}-CH₃ (I),
où x est un nombre entier allant de 1 à 8, et y est un nombre entier allant de 2 à 10, dans une émulsion selon une ou plusieurs des revendications 1 à 8, le procédé comprenant les étapes du processus suivantes consistant à :
- émettre un champ magnétique alternatif,
- détecter une absorption du champ magnétique émis, par un échantillon à examiner,
**caractérisé par** les étapes du processus suivantes consistant à :
- superposer au moins une image de proton de fluor ¹⁹F sur une image de proton de ¹H,
par lesquelles les protons de l'image de ¹H sont affichés comme image en échelle de gris, et les protons de l'image de fluor ¹⁹F sont affichés en utilisant un dégradé de couleur, une extrémité dudit dégradé de couleur indiquant une faible concentration de ¹⁹F, et l'autre extrémité dudit dégradé de couleur indiquant une forte concentration de ¹⁹F.

14. Procédé selon la revendication 13, **caractérisé par** les étapes du processus suivantes consistant à :
- éliminer des signaux perturbateurs générés par des protons de fluor ¹⁹F dans un second échantillon à ne pas analyser, par les étapes du processus suivantes :
- comparer la valeur de la concentration en fluor dans un premier pixel avec les valeurs de tous les pixels dans l'espace tridimensionnel autour du premier pixel,
par lequel lesdits deux pixels sont considérés comme appartenant à un regroupement de pixels, si la différence entre les valeurs de la concentration en fluor de deux pixels adjacents est inférieur à une valeur de seuil,
- enlever ledit regroupement de pixels perturbateur de l'image obtenue, si le regroupement ne représente pas l'échantillon à analyser.

15. Procédé, comprenant l'étape consistant à acquérir des procédés d'imagerie non invasives chez un patient auquel l'émulsion selon une ou plusieurs des revendications 1 à 8 a été administrée préalablement afin de la dissoudre dans la circulation sanguine du patient.
